# EUROPEAN PATENT APPLICATION

(11) **EP 1 388 589 A1**
(43) Date of publication of application: **11.02.2004**
(21) Application number: 02017758.0
(22) Date of filing: 09.08.2002
(51) Int. Cl.: C12Q 1/68

(54) **Genetic polymorphisms sensitively predicting adverse drug reaction (ADR) and drug efficacy**

(71) Applicant: Bayer HealthCare AG, 51368 Leverkusen (DE)
(72) Inventor: Schwers, Stephan, Dr., 51145 Köln (DE); Kallabis, Harald, Dr., 51061 Köln (DE); Reifenberger, Elke, Dr., 53757 St.-Augustin-Hangelar (DE); Stropp, Udo, Dr., 42781 Haan (DE)

(57) **Abstract**

The invention provides diagnostic methods and kits including oligo and/or polynucleotides or derivatives, including as well antibodies determining whether a human subject is at risk of getting adverse drug reaction after statin therapy or whether the human subject is a high or low responder or a good a or bad metabolizer of statins. The invention provides further diagnostic methods and kits including antibodies determining whether a human subject is at risk for a cardiovascular disease. Still further the invention provides polymorphic sequences and other genes.

The present invention further relates to isolated polynucleotides encoding a phenotype associated (PA) gene polypeptide useful in methods to identify therapeutic agents and useful for preparation of a medicament to treat cardiovascular disease or influence drug response, the polynucleotide is selected from the group comprising:
SEQ ID 1-21 with allelic variation as indicated in the sequences section contained in a functional surrounding like full length cDNA for PA gene polypeptide and with or without the PA gene promoter sequence.

Sequences:

SEQUENCE LISTING

<110>  Bayer AG

<120>  Genetic Polymorphisms sensitively predicting Adverse Drug
Reactions (ADR) and Drug Efficacy

<130>  LeA

<160>  21

<170>  PatentIn version 3.1

<210>  1
<211>  587
<212>  DNA
<213>  Homo Sapiens

<220>
<221>  variation
<222>  (195)..(195)
<223>  baySNP160_C195T


<400>  1
tggagcctcc ccataggcac tcggggcctc ccctgggatg agactccagc tttgctccct
60

gccttcctcc cccaggcatg gctggggact ggaagaccac cttcaccgtg gcgcagaatg
120

agcgcttcga tgcggactat gcggagaaga tggcaggctg cagcctcagc ttccgctctg
180

agctgtgaga ggggytcctg gagtcactgc agagggagtg tgcgaatcaa gcctgaccaa
240

gaggctccag aataaagtat gatttgtgtt caatgcagag tctctattcc aagccaagag
300

aaaccctgag ctgaaagagt gatcgcccac tggggccaaa tacggccacc tccccgctcc
360

agctcctcaa cttgccctgt ttggagaggg gagagggtct ggagaagtaa aacccaggag
420

acgagtagag ggggaatgtg tttaatccca gcacgtcctc tgctgtcctg ccctgtgtcg
480

ttgggggatg gcgagtctgc caggcggcat cactttttct tgggttcctt acaagccacc
540

acgtatctct gagccacatt gaggggaggg gaatagccat ctgcata
587


<210>  2
<211>  596
<212>  DNA
<213>  Homo Sapiens

<220>
<221>  variation
<222>  (168)..(168)
<223>  baySNP1278_A168G


<400>  2
tgtcaaatct atctcctccc actacagata gaggggtggg ggtgaggcag cacaggaggc
60

agagaggcga ggtgcccagg cagcccgaag cagggatgtg ctggacgctg cccagcagga
120

tggttccaga ccgagctgga ggggagttcg gccggccaga gcaagctrag gagctctgga
180

cggccagccc cggaacccag agggctgtta ggtggccagg ctgtggaaga ggaggggctc
240

tggcgatacc ttttctgttg ccataggaag tctcttagac aaaatgaaag ctccctcaac
300

ctgtcatctc aatatctgtt tctgtgagag tatttggttt ttcagaaatg tatgggccag
360

aaaaattctc tcattcaaca ggcatttatt gagtgcctcc tacgttccag gcactatgcc
420

aaagctaagt aaaacccaag agggcttttc tttgaccagg atctgagtca ggactacagc
480

atgtaagctt tctattacat gtcttctaaa tcaagtgaaa ccagaaagac caaaacatgc
540

ttaagagtaa agatcagact tctcgttctt tgaaaacatc taacacctta gagtta
596


<210>  3
<211>  784
<212>  DNA
<213>  Homo Sapiens

<220>
<221>  variation
<222>  (507)..(507)
<223>  baysNP1371_C507T


<400>  3
ctgccctcac attaacagag cttccctcgc tcctctcctc tcctctcttt gggtcttccc
60

ttccttacca ccttctgatt cactccctgc agtcttcact tacttctaca aggggaacaa
120

atactggaaa ttcaacaacc agaagctgaa ggtagaaccg ggctacccca agtcagccct
180

gagggactgg atgggctgcc catcgggagg ccggccggat gaggggactg aggaggagac
240

ggaggtgatc atcattgagg tggacgagga gggcggcggg gcggtgagcg cggctgccgt
300

ggtgctgccc gtgctgctgc tgctcctggt gctggcggtg ggccttgcag tcttcttctt
360

cagacgccat gggaccccca ggcgactgct ctactgccag cgttccctgc tggacaaggt
420

ctgacgccca ccgccggccc gcccactcct accacaagga ctttgcctct gaaggccagt
480

ggcagcaggt ggtggtgggt gggctgytcc catcgtcccg agccccctcc ccgcagcctc
540

cttgcttctc tctgtcccct ggctggcctc cttcaccctg accgcctccc tccctcctgc
600

cccggcattg catcttccct agataggtcc cctgagggct gagtgggagg gcggcccttt
660

ccagcctctg cccctcaggg gaaccctgta gctttgtgtc tgtccagccc catctgaatg
720

tgttgggggc tctgcacttg aagcaggacc cttaaacctt gctggtaaag tcaaaagggg
780

tcat
784


<210>  4
<211>  752
<212>  DNA
<213>  Homo Sapiens

<220>
<221>  variation
<222>  (201)..(201)
<223>  baySNP1806_A201G


<400>  4
ctgggggttt gggacagaac acacgcagct tcagtcagtt ggtaaacggg tccctttcct
60

ctggggcaga aacgctttgg ggtttgactc aaatcatgga ctccttgggg gcctattctt
120

cgggctaact ctttgcatgt tctgcaggga gccaaacaag ccgctcctac acttcgctgg
180

tcctgggcgt cctggtgggc rgtttcatca ttgctggtgt ggctgtaggc atcttcctgt
240

gcacaggtgg acggcgatgt taattactct ccagccccgt cagaaggggc tggattgatg
300

gaggctggca agggaaagtt tcagctcact gtgaagccag actccccaac tgaaacacca
360

gaaggtttgg agtgacagct cctttcttct cccacatctg cccactgaag atttgaggga
420

ggggagatgg agaggagagg tggacaaagt acttggtttg ctaagaacct aagaacgtgt
480

atgctttgct gaattagtct gataagtgaa tgtttatcta tctttgtgga aaacagataa
540

tggagttggg gcaggaagcc tatggcccat cctccaaaga cagacagaat cacctgagcg
600

ttcaaaagat ataaccaaat aaacaagtca tccacaatca aaatacaaca ttcaatactt
660

ccaggtgtgt cagacttggg atgggacgct gatataatag ggtagaaagg agtaacacga
720

agaagtggtg gaatgtaaaa tccagtcata tg
752

<210> 5
<211> 1245
<212> DNA
<213> Homo Sapiens

<220>
<221> variation
<222> (719)..(719)
<223> baySNP2178_C719T

<400> 5
tgcataaagc tccagattca tttccttcag gtgatcacgt gctctgaatt ccaatcaaca
60

ctggttctat cttgttctgg ggtttaatcc agtctctaga gctgggctag caagttgatt
120

ttccccagcc agacaaatgg cagggacagt taccagctgt caggacaatg cctgcctctt
180

ttcaattctg agctgccaaa gcagcacccc tcttttagaa acctaagcct ctgtaacacc
240

agcatccgaa tcccaccatc catctttggt ctccaattcc atagtaaatg gtataagtcc
300

cctgacatct ggctgatgct acaagctcaa taaggagcct ccaaagtcct gtcacttctg
360

ctttctaact gctcgaatgt tccctaaggg ccaggctcca ggcactatat ctccaccagt
420

cccataggac ctgtggtcca atgaggcttc agtctagccc tcatatttgc aaaaagaatg
480

cttggggtcc aacattattt gtcctagagc tggaggttgt gtgacattag aaacatgttg
540

ataagcagga aatagaaggg gaggaacctc agagggaaag gatgtccagc ctgtgtgcct
600

gctgcctgca aaaccagctg ggaggaacag tgacagggaa catcggaaat atcccactag
660

cccactccag ctaaacgggt atcctgagcc ctgacttggt ggctcattac caatctctyc
720

tggaacgtgt ttgccatgga agctgaagca ggtggtgacg ttcaggcagt tcacaggctg
780

ctgtccgtcg tgacactgag gcgctgtgat gttgatggag cccgggagga agatggagac
840

atccaccgta atgacaggcc ttgctctgca gggcaagaga ggaaggtgac ttagtcgtgg
900

taaggagcag ctctcagaac caaacaagaa ctagaccaac aggataaatc ctaggccttc
960

aacccagcat ttaattaaag catgaaatat cccaagagag aagggctagt atgttctgtg
1020

agcatttttt tctggtttta gaaggggcat attgcctagt aaattaatag ttagcgtccc
1080

aataataaaa gcaagtttgt tgttaataaa acgcatctgg ttgaagccat aaatgaacca
1140

ccagatgctg ggtaattatt taaaatttta ccaattaagt atcaatttcc atattattag
1200

ggagataaga atacatttaa gaggatttcc tagagcagat gccat
1245


<210> 6
<211> 645
<212> DNA
<213> Homo Sapiens

<220>
<221> variation
<222> (548)..(548)
<223> baySNP2198_C548T


<400> 6
actggatatg taacaaacct tacaaataat aaggaaacat gttcacttat tgactattat
60

agaatggaac tcaaggaaat ctgtgtcagt ggatgctgct ctgtggtccg aagtcttcca
120

tagagacttt gtgaaaaaaa attttatagt gtcttgggaa ttttcttcca aacagaacta
180

tggaaaaaaa ggaagaaatt ccaggaaaat ctgcactgtg ggcttttatt gccatgagct
240

agaagcatca caggttgacc aataaccatg cccaagaatg agaagaatga ctatgcaacc
300

tttggatgca ctttatatta ttttgaatcc agaaataatg aaataactag gcgtggactt
360

actatttatt gctgaatgac taccaacagt gagagccctt catgcatttg cactattgga
420

aggagttaga tgttggtact agatactgaa tgtaaacaaa ggaattatgg ctggtaacat
480

aggtttttag tctaattgaa tcccttaaac tcagggagca tttataaatg gacaaatgct
540

tatgaaayta agatttgtaa tatttctctc tttttagaga aatttgccaa tttactttgt
600

tatttttccc caaaaagaat gggatgatca tgtatttatt ttttt
645

<210> 7
<211> 544
<212> DNA
<213> Homo Sapiens

<220>
<221> variation
<222> (208)..(208)
<223> baySNP2214_A208T

<400> 7
agcaagctac cgtctggaca actggccacc atctcagaac gccattgacc ctctgaggca
60

agccattcgg ctgaatcctg acaaccagta ccttaaagtc ctcctggctc tgaagcttca
120

taagatgcgt gaagaaggtg aagaggaagg tgaaggagag aagttagttg aagaagcctt
180

ggagaaagcc ccaggtgtaa cagatgtwct tcgcagtgca gccaagtttt atcgaagaaa
240

agatgagcca gacaaagcga ttgaactgct taaaaaggct ttagaataca taccaaacaa
300

tgcctacctg cattgccaaa ttgggtgctg ctatagggca aaagtcttcc aagtaatgaa
360

tctaagagag aatggaatgt atgggaaaag aaagttactg gaactaatag gacacgctgt
420

ggctcatctg aagaaagctg atgaggccaa tgataatctc ttccgtgtct gttccattct
480

tgccagcctc catgctctag cagatcagta tgaagacgca gagtattact tccaaaagga
540

attc
544

<210> 8
<211> 585
<212> DNA
<213> Homo Sapiens

<220>
<221> variation
<222> (490)..(490)
<223> baySNP2267_C490T

<400> 8
taataaatac ttggtattta cctatctacc tcccagtcca ggggatggga ggacaggagc
60

tttttctcac tgctacaggc aggagaatga aactcagaaa tgcagggaag gacacagagg
120

cctggggctc cgttctgctc ttccctgtct ttaaatatga ggcttaaata gacataaata
180

ttaaatagag ataaatacat gggatgggga gggcttggct cagggctggg caaggtggcg
240

tagaacgcgg tacgacacct ccaggaagct ctgcagatgg gaggcaacca ggacccctcc
300

tgcccggcgc tggaaagcag aggcgaaggc cggcatggca ccctgggtgg gctgcagggc
360

aggggccatt cccagttctt ccatctgtgg gggaaagctg tgatcagtgg ttgggccctt
420

ctgagtgggc gttattacaa atgaggaaat gctgggggtg acagcatgga cagggcccat
480

acacaggccy ggctgtggtg cccagaatgc cagcacgacc ttggccaccc tgcccaacaa
540

ggctcacctg ctgccagatg gtggtggcaa agtcggcgac gtcca
585

<210> 9
<211> 714
<212> DNA
<213> Homo Sapiens

<220>
<221> variation
<222> (194)..(194)
<223> baySNP3907_A194C

<400> 9
ctgcagatgc tctaatgtct tctggcgggc acggtgttga tcggccagcc ggtaagccac
60

tgctgtcacc atggctgcac ctttgccact gccatcctcg gagcggagga agcggacatc
120

gcagccgggc accagccgcc gcacggtctt atgtagacgc ttggcaaaac tgcagttccc
180

cagggcgggt aagmgacagg tgtccatggt tagggtcaat ggtgcaaata gttagacagt
240

tgcccaccgc cttaatccaa atgccaaaag gcacctgcag gagtgggtgc tgccctaaac
300

atcctctctc cactgtccca agttaggaac cactacaaga accatatcaa ctatgaccac
360

ccaggattgg ttcacccccg ccaatgaaag gaaaagacct tttaataagc cactgtcccc
420

tacatgcagc cacacaagac ctcctcttcc tcactgaaga aggccccctg gccactgctc
480

ccttgcccaa catggtacca tcagcctctt tgatggagca gccctgtggt attaggtggt
540

tctgatgcca cctatctgtc ttgttctatc actcatttca ctagctgctg tctgggaaag
600

acccaaaatt aaaagctaga gatgctcttc tggactttag cctctctttt gcagtttctg
660

ccaatgatcc ccaaagtgtc tgcacaggct aagacacgtg ttcagcagga ctct
714


<210>  10
<211>  582
<212>  DNA
<213>  Homo Sapiens

<220>
<221>  variation
<222>  (446)..(446)
<223>  baySNP4564_A446G


<400>  10
agaatccatg acttcccttc atgctgggaa gttatttctt ttatcactgt tttccaaact
60

tcagccattt gaatatcact atcatgattt ttgccatata ttaacatgaa acaattgctt
120

ttctttaaaa cagctcagaa aaccaaatat aacatctctc ctgttgtact ttcaatcatt
180

tccccactga attgaaaatc catcctaagc agagatgcta tcattttgga gagctgtgtg
240

gagcgtggac aagaactctg ctccatattc cccctgctcc caaacaaaac aaaatatgaa
300

accaaccagg aaacaccact cctaatattg caggaacgaa aggcctctta cctggacttc
360

tgcaaaagag atagcagagg tactgaggct gcgggggccg gaagccgcaa ggaagaggta
420

gtgtgggcga cagcagcacg gggctrctgg gaataggagc cgtgtggtca gcggtttccc
480

aagccattgc gagggccctg cgacgctgtg gacctgagcg tgtaaaagga tgatggagcc
540

cctttaccgg gagcagaagt cctcgcgtgg gtgcaaacgg ga
582


<210>  11
<211>  626
<212>  DNA
<213>  Homo Sapiens

<220>
<221>  variation
<222>  (157)..(157)
<223>  baysNP4869_A157G


<400>  11
tgtttcctag tttccagggt gtagatattt ccatttggcc aatttcaagc tcctaatatg
60

ataccaatga acagagattt gggaagccat gtgtacaata tattctcatc aatcacacaa
120

cccacctctg ttgctcatgg tcactctttt tgtagtrgct tcaactgtac acctacctca
180

acatttcatt catatttctt tactactttg aaactatgtt gctaaaatca ggtaagcaaa
240

cctgacattg ggccttaaat atcttatgaa atattggagg agtggggtta aattaggtta
300

tataattcca aagaaagttc atgagacaaa ttactccatt tctggttggt ggagttcata
360

taaagttttc cagactactt agtttggctc aaggaccaaa aagaaaggag gatgggagcc
420

attacttact caattgtgtt tctgtccact tttcctgtca tgttaatgcc atagaactgc
480

tgcatggcag ctagggcaga ctgcatggtc tctgcagagc gcagcactga cattctgggg
540

tcagtcggtg gaaggtagcc gtacttttgt aaccaaacct gcaataacaa gtacagtttc
600

ttttagatca attttacaat ttaaca
626

<210>  12
<211>  1217
<212>  DNA
<213>  Homo Sapiens

<220>
<221>  variation
<222>  (605)..(605)
<223>  baySNP5569_A605G


<400>  12
tgcatacctg gtgggaggaa aagagttggg aacttcagat ggacctagta cccactgaga
60

tttccacgcc gaaggacagc gatgggaaaa atgcccttaa atcataggaa agtatttttt
120

taagctacca attgtgccga gaaaagcatt ttagcaattt atacaatatc atccagtacc
180

ttaagccctg attgtgtata ttcatatatt ttggatacgc accccccaac tcccaatact
240

ggctctgtct gagtaagaaa cagaatcctc tggaacttga ggaagtgaac atttcggtga
300

cttccgcatc aggaaggcta gagttaccca gagcatcagg ccgccacaag tgcctgcttt
360

taggagaccg aagtccgcag aacctgcctg tgtcccagct tggaggcctg gtcctggaac
420

tgagccgggg ccctcactgg cctcctccag ggatgatcaa cagggcagtg tggtctccga
480

atgtctggaa gctgatggag ctcagaattc cactgtcaag aaagagcagt agaggggtgt
540

ggctgggcct gtcaccctgg ggccctccag gtaggcccgt tttcacgtgg agcatgggag
600

ccacraccct tcttaagaca tgtatcactg tagagggaag gaacagaggc cctgggccct
660

tcctatcaga aggacatggt gaaggctggg aacgtgagga gaggcaatgg ccacggccca
720

ttttggctgt agcacatggc acgttggctg tgtggccttg gcccacctgt gagtttaaag
780

caaggcttta aatgactttg gagagggtca caaatcctaa aagaagcatt gaagtgaggt
840

gtcatggatt aattgacccc tgtctatgga attacatgta aaacattatc ttgtcactgt
900

agtttggttt tatttgaaaa cctgacaaaa aaaaagttcc aggtgtggaa tatgggggtt
960

atctgtacat cctggggcat taaaaaaaaa atcaatggtg gggaactata aagaagtaac
1020

aaaagaagtg acatcttcag caaataaact aggaaatttt tttttcttcc agtttagaat
1080

cagccttgaa acattgatgg aataactctg tggcattatt gcattatata ccatttatct
1140

gtattaactt tggaatgtac tctgttcaat gtttaatgct gtggttgata tttcgaaagc
1200

tgctttaaaa aaataca
1217

<210> 13
<211> 566
<212> DNA
<213> Homo Sapiens

<220>
<221> variation
<222> (254)..(254)
<223> baySNP6872_A254G

<400> 13
tgctcatcaa ggcagaccaa catgtgcaag gggaaaaaat tttaaggcct catctgttat
60

atggtcaaga agacaatcat attaggcctt cccaaaaatg ttcaagtgac agaataaatg
120

gctataaggc caacatatta attactgagc ttttaagaaa tggtcactgt aactaggaag
180

cagaatatag gcccagtaga aatacacaca tgcatgcaca ttgaaataag acaagaaaga
240

tacctaaata acargctggt gctacaaaaa tgaagaaaaa tactagcacc aaatggaaca
300

aacacatttt gaagtgatag attctcatgg tatgtctacc caaagaccaa acagcactcc
360

tgcagacccc ctttgctctc cttcaggatt ctattaatga agcattttac agcataaaaa
420

agtcaaccat accagctgat tcaaagtatc ccaaggcctc ctgagcaggc ccgtggaaca
480

taagtcttcc tgaggccaat aaggtgaggc tatcaaacaa cttgaagatg gaatatcgag
540

gctgatgaat ggagaagatg attgtt
566

<210> 14
<211> 1043
<212> DNA
<213> Homo Sapiens

<220>
<221>  variation
<222>  (889)..(889)
<223>  baysNP7372_C889T


<400>  14
atccccttca gcctcctgaa tagctgggac tacaggcaca ggctaccatg cttagctaat
60

ttttaaagtt tgtgcagagg cagcttcact gtgttgccca ggctggtctt gaactcctgg
120

gctcaagcaa ttcacccgcc ttggcctccc aaagtgctgg gatgatagac atgagccacc
180

atgctcggca gtgttcatct ttcgtagcta ttgctagacc atttcctgaa tgaaatgctt
240

ataccagtat ccactttacc agcagtgtcc tgtgtcttgc agctccacag tcttaccaac
300

atttgctgtt ggccaagtga atgggtagaa aatggaatct ttgggaactt ggtttctagg
360

tccctgagcc ctgctgctga atatctcctc ttatgtttat tggcatatgt gtttcctgtg
420

aaatattcat gtcttttgcc agtttttcat tagggttctt ggtgctttat tttttgttga
480

tctatagggc tctttatata ttgctgattt taatccttcg tgtctattgc aaatatcttc
540

tcccagcttt taacctatta ttcctctttc tttaaggtgt cttctgttga tcagccattc
600

ttcattttaa catagcgaaa tgtatttttt ccctagtact cagttttctg ggtctggttt
660

gaagtctttt cctgaacata acccttgcct ttaaggagcg tatgcattct ccagtggagg
720

agacagataa tggcactgag acgtcatgtg ggcattgatt gttagaagtg gccacttctg
780

cctgtggagc gggagcgaac ttaggaaaga tctcacaggg cagagtcttg agctaggaat
840

taagagatga gtaagtggac aagtgcgatg ggcattctca gtgggggayg aagggcatga
900

aacatcctga cctgccctgg gcactgccag gaggtccaga tggcaggagc tgacggtttg
960

aggggctcgg gcagggcatc ccgaggacag ctttgggtga tctgtatgca agggaatatt
1020

gaagccatgg gaacagatcc tga
1043

<210>  15
<211>  604
<212>  DNA
<213>  Homo Sapiens

<220>
<221>  variation
<222>  (262)..(262)
<223>  baySNP8242_A262G


<400>  15
tcttttctct ctgccttttc cccaagggcc aggacccctg gatctctggg cagagcagac
60

gcaggcccct ataatagccc tcatgctaga aaggagccgg agcctgtgta taaggccagc
120

gcagcctact ctggacagtg cagggttccc actctcccaa ctccccatct gcttgcctcc
180

agacccacat tcacacacga gccactgggt tggaggagca tctgtgagat gaaacaccat
240

tctttcctca atgtctcagc trtctaactg tgtgtgtaat caggccaggt cctccctgct
300

gggcagaaac catgggagtt aagagattgc caacatttat tagaggaagc tgacgtgtaa
360

cttctctgag gcaaaattta gccctccttt gaacaggaat ttgactcagt gaaccttgta
420

cacactcgca ctgagtctgc tgctgatgat actgtgcacc ccactgtctg ggttttaatg
480

tcaggctgtt cttttaggta tggcggcttt tccctgggtg tcagtaatac tcaagcactt
540

cctccgagtc aagaagttaa tgatgccatc aaacaaatga agaaacacct aaagctggcc
600

aagg
604


<210>  16
<211>  576
<212>  DNA
<213>  Homo Sapiens

<220>
<221>  variation
<222>  (378)..(378)
<223>  baySNP8589_C387T


<400>  16
aattcctgac ctcaggtgat ctcagcccgc ttcagcctcc caaagtgctg ggataacagg
60

catgagccgc tgtgcctggc ctgagcctat tttcttagca gttgaacaat aacagtacct
120

acttcacaga gttgctgaga aaattgagag aaaatttagg taacatcctt agtagacagg
180

cccttctgcc aatcaagtcc atttatttat ggcccacaat gtacctcctc tatgccacca
240

catgtcaaga ccaggattca ggaaatggtg ctttcttgtt gtttggtttg ttgtttttac
300

ctgtctcttt gatgagttcc aagcatccct taggcgtaca aggaatgaaa cagtcattga
360

ggtcacctct agcaagtytc ccagcattga tgctagtcaa tcttaaaaaa gtgatggaaa
420

taagaaggaa ataaaggcaa ttagatgtct ttactcttta gaaacattta tgctgtattc
480

cccaaataga aacatattat aaatgaaagc atgcaatgag acataggaag ccaagccaca
540

cttacccatc cacatccttc tcgggtgcaa tagcat
576


<210> 17
<211> 557
<212> DNA
<213> Homo Sapiens

<220>
<221> variation
<222> (64)..(64)
<223> baySNP10771_C64G


<400> 17
ggaggttagg gtccccaggg aagagcagaa cctgggccca ccgagttact gaaggcaagg
60

aagsgtggcc tgtgtcaccc tggcggccct gcctgggtct gctcctcgct gtgcacactg
120

catcacgccc cgctggctgc ctccctctgc ctcttcccgt acagacgcac actcacagag
180

atcatgcaca cgacacgggg cggggacgga gggcccatgg cagagaaggg agcatggggg
240

ccgggaggga gggtgctctg agccagaggc gggggttggg agggcagccc agccaggtgg
300

cagcagccag ggcctccttg gagtggccca cctggagtgg agtggcagtt tgggtggctt
360

ctaggcgaag atgcccccaa cagtggaggc gatgacgatg cccaggatca cacagcagat
420

gatgatcatg attctcttct gcatgggaag cgggcaagag aggcctcata cagtgttggc
480

ggcacgtggg gtgggactat tgcaaaggct gatgactgga cagtcggagg ggatccaagc
540

aagggaaggt gactgcc
557


<210>  18
<211>  583
<212>  DNA
<213>  Homo Sapiens

<220>
<221>  variation
<222>  (55)..(55)
<223>  baySNP12399_A55G


<400>  18
tgcgtgtgtt tcgcaggagg agcaccgtga ggccaagctg accctgcggc ccccragcct
60

ggcagccccc tacgccccag tgcagagttg gcaacaccag ccagagaaac tcatcttcga
120

gtcctgtggt tatgaagcca atgtgagttg ctcccaccct cccagcaggg ccggcctccc
180

tgctccttct cggccaggca cctgcggcca agtcccagca gggactccac aaagccaggc
240

ccagggcttt tgagctgttc tccactctca gccactcttg acagctaagg cgaaggggtg
300

gaggcctctg gatcctttag ctagggagtt gaatggaggg tctctccagc tggggcagga
360

gagggggtgt tgttattgag aggggtaact acttgattgt gggacagtcc caaattggtt
420

gggtcacccc tgcatggaaa cgcaggattt cctaagcctg gccactgctc gcccccacag
480

tatctgggct gccatgctga tcaaagatct gcgagggaca gaatccacgc aagacgcctg
540

tgccaagatg cgggtagggt gcctgtgtgg gctggagggc gct
583


<210>  19
<211>  567
<212>  DNA
<213>  Homo sapiens

<220>
<221>  variation
<222>  (331)..(331)
<223>  baySNP8164_A331T

<400>  19
tacgtggatt ttgttgtttt tgtatcccag ttcagacaca agcactttat tcgttcattc
60

ttccatcagg attcacaagt aaatcacaca aatgggcatc acacttaccc ctataatctt
120

tgaaaatatt attgcaaatg ctggttgcag gcctccattt ataatggcac aaaatacacc
180

aacaacaaaa taaggccatt cagttaaatt tagcttcata atcctccaaa aggaaactgg
240

aggtatactt tcatcctaga aaacacaaat tattacaaca ggctagttaa aaacttttat
300

atgtacaatt cttacatacg cacaaaaatt wgtaaaggaa tatatcctat ccttattcct
360

tatcaatgaa taagtgatga caattttgct ttaaaaaatg tcatagtgat tattatttag
420

cttcaaaaaa atgttgacaa attttgctac aatactcctt tattttttaa aaaataaatg
480

atgtttccac aaacacttac ttttatttgg tactcagaac ctgacaagag tatttataag
540

caggagtaaa cagaatggaa attttga
567


<210>  20
<211>  900
<212>  DNA
<213>  Homo sapiens

<220>
<221>  variation
<222>  (312)..(312)
<223>  baySNP900066_C312T


<400>  20
caggtttgtg gggggaaaac aaaagcagcc cattacccag aggactgtcc gccttcccct
60

caccccagcc taggcctttg aaaggaaaca aaagacaaga caaaatgatt ggcgtcctga
120

gggagattca gcctagagct ctctctcccc caatccctcc ctccggctga ggaaactaac
180

aaaggaaaaa aaaattgcgg aaagcaggat ttagaggaag caaattccac tggtgccctt
240

ggctgccggg aacgtggact agagagtctg cggcgcagcc ccgagcccag cgcttcccgc
300

gcgtcttagg cyggcgggcc cgggcggggg aaggggacgc agaccgcgga ccctaagaca
360

cctgctgtac cctccacccc caccccaccc cacccacctc cccccaactc cctagatgtg
420

tcgtgggcgg ctgaacgtcg cccgtttaag gggcgggccc cggctccacg tgctttctgc
480

tgagtgactg aactacataa acagaggccg ggaasggggc ggggaggagg gagagcacag
540

gctttgaccg atagtaacct ctgcgctcgg tgcagccgaa tctataaaag gaactagtcc
600

cggcaaaaac cccgtaattg cgagcgagag tgagtggggc cgggacccgc agagccgagc
660

cgacccttct ctcccgggct gcggcagggc agggcgggga gctccgcgca ccaacagagc
720

cggttctcag ggcgctttgc tccttgtttt ttccccggtt ctgttttctc cccttctccg
780

gaaggcttgt caaggggtag gagaaagaga cgcaaacaca aaagtggaaa acaggtaaga
840

ggctctccag tgacttactt gggcgttatt gttttgtttc gaggccaagg aggcttcggg
900


<210>  21
<211>  900
<212>  DNA
<213>  Homo sapiens

<220>
<221>  variation
<222>  (515)..(515)
<223>  baySNP900073_C515G


<400>  21
caggtttgtg gggggaaaac aaaagcagcc cattacccag aggactgtcc gccttcccct
60

caccccagcc taggcctttg aaaggaaaca aaagacaaga caaaatgatt ggcgtcctga
120

gggagattca gcctagagct ctctctcccc caatccctcc ctccggctga ggaaactaac
180

aaaggaaaaa aaaattgcgg aaagcaggat ttagaggaag caaattccac tggtgccctt
240

ggctgccggg aacgtggact agagagtctg cggcgcagcc ccgagcccag cgcttcccgc
300

gcgtcttagg cyggcgggcc cgggcggggg aaggggacgc agaccgcgga ccctaagaca
360

cctgctgtac cctccacccc caccccaccc cacccacctc cccccaactc cctagatgtg
420

tcgtgggcgg ctgaacgtcg cccgtttaag gggcgggccc cggctccacg tgctttctgc
480

tgagtgactg aactacataa acagaggccg ggaasggggc ggggaggagg gagagcacag
540

gctttgaccg atagtaacct ctgcgctcgg tgcagccgaa tctataaaag gaactagtcc
600

cggcaaaaac cccgtaattg cgagcgagag tgagtggggc cgggacccgc agagccgagc
660

cgacccttct ctcccgggct gcggcagggc agggcgggga gctccgcgca ccaacagagc
720

cggttctcag ggcgctttgc tccttgtttt ttccccggtt ctgttttctc cccttctccg
780

gaaggcttgt caaggggtag gagaaagaga cgcaaacaca aaagtggaaa acaggtaaga
840

ggctctccag tgacttactt gggcgttatt gttttgtttc gaggccaagg aggcttcggg
900

## Description

### Technical Field

This invention relates to genetic polymorphisms useful for assessing cardiovascular risks in humans, including, but not limited to, atherosclerosis, ischemia/reperfusion, hypertension, restenosis, arterial inflammation, myocardial infarction, and stroke. In addition it relates to genetic polymorphisms useful for assessing the response to lipid lowering drug therapy. Specifically, the present invention identifies and describes gene variations which are individually present in humans with cardiovascular disease states, relative to humans with normal, or non-cardiovascular disease states, and/or in response to medications relevant to cardiovascular disease. Further, the present invention provides methods for the identification and therapeutic use of compounds as treatments of cardiovascular disease. Moreover, the present invention provides methods for the diagnostic monitoring of patients undergoing clinical evaluation for the treatment of cardiovascular disease, and for monitoring the efficacy of compounds in clinical trials. Still further, the present invention provides methods to use gene variations to predict personal medication schemes omitting adverse drug reactions and allowing an adjustment of the drug dose to achieve maximum benefit for the patient. Additionally, the present invention describes methods for the diagnostic evaluation and prognosis of various cardiovascular diseases, and for the identification of subjects exhibiting a predisposition to such conditions.

### Background of the Invention

Cardiovascular disease is a major health risk throughout the industrialized world.

Cardiovascular diseases include but are not limited by the following disorders of the heart and the vascular system: congestive heart failure, myocardial infarction, atherosclerosis, ischemic diseases of the heart, coronary heart disease, all kinds of atrial and ventricular arrhythmias, hypertensive vascular diseases and peripheral vascular diseases.

Heart failure is defined as a pathophysiologic state in which an abnormality of cardiac function is responsible for the failure of the heart to pump blood at a rate commensurate with the requirement of the metabolizing tissue. It includes all forms of pumping failure such as high-output and low-output, acute and chronic, right-sided or left-sided, systolic or diastolic, independent of the underlying cause.

Myocardial infarction (MI) is generally caused by an abrupt decrease in coronary blood flow that follows a thrombotic occlusion of a coronary artery previously narrowed by arteriosclerosis. MI prophylaxis (primary and secondary prevention) is included as well as the acute treatment of MI and the prevention of complications.

Ischemic diseases are conditions in which the coronary flow is restricted resulting in an perfusion which is inadequate to meet the myocardial requirement for oxygen. This group of diseases include stable angina, unstable angina and asymptomatic ischemia.

Arrhythmias include all forms of atrial and ventricular tachyarrhythmias (atrial tachycardia, atrial flutter, atrial fibrillation, atrio-ventricular reentrant tachycardia, preexitation syndrome, ventricular tachycardia, ventricular flutter, ventricular fibrillation) as well as bradycardic forms of arrhythmias.

Hypertensive vascular diseases include primary as well as all kinds of secondary arterial hypertension (renal, endocrine, neurogenic, others).

Peripheral vascular diseases are defined as vascular diseases in which arterial and/or venous flow is reduced resulting in an imbalance between blood supply and tissue oxygen demand. It includes chronic peripheral arterial occlusive disease (PAOD), acute arterial thrombosis and embolism, inflammatory vascular disorders, Raynaud's phenomenon and venous disorders.

Atherosclerosis, the most prevalent of vascular diseases, is the principal cause of heart attack, stroke, and gangrene of the extremities, and thereby the principal cause of death. Atherosclerosis is a complex disease involving many cell types and molecular factors (for a detailed review, see Ross, 1993, Nature 362: 801-809 and Lusis, A. J., Nature 407, 233-241 (2000)). The process, in normal circumstances a protective response to insults to the endothelium and smooth muscle cells (SMCs) of the wall of the artery, consists of the formation of fibrofatty and fibrous lesions or plaques, preceded and accompanied by inflammation. The advanced lesions of atherosclerosis may occlude the artery concerned, and result from an excessive inflammatory-fibroproliferative response to numerous different forms of insult. For example, shear stresses are thought to be responsible for the frequent occurrence of atherosclerotic plaques in regions of the circulatory system where turbulent blood flow occurs, such as branch points and irregular structures.

The first observable event in the formation of an atherosclerotic plaque occurs when blood-borne monocytes adhere to the vascular endothelial layer and transmigrate through to the sub-endothelial space. Adjacent endothelial cells at the same time produce oxidized low density lipoprotein (LDL). These oxidized LDLs are then taken up in large amounts by the monocytes through scavenger receptors expressed on their surfaces. In contrast to the regulated pathway by which native LDL (nLDL) is taken up by nLDL specific receptors, the scavenger pathway of uptake is not regulated by the monocytes.

These lipid-filled monocytes are called foam cells, and are the major constituent of the fatty streak. Interactions between foam cells and the endothelial and SMCs which surround them lead to a state of chronic local inflammation which can eventually lead to smooth muscle cell proliferation and migration, and the formation of a fibrous plaque. Such plaques occlude the blood vessel concerned and thus restrict the flow of blood, resulting in ischemia.

Ischemia is a condition characterized by a lack of oxygen supply in tissues of organs due to inadequate perfusion. Such inadequate perfusion can have number of natural causes, including atherosclerotic or restenotic lesions, anemia, or stroke, to name a few. Many medical interventions, such as the interruption of the flow of blood during bypass surgery, for example, also lead to ischemia. In addition to sometimes being caused by diseased cardiovascular tissue, ischemia may sometimes affect cardiovascular tissue, such as in ischemic heart disease. Ischemia may occur in any organ, however, that is suffering a lack of oxygen supply.

The most common cause of ischemia in the heart is atherosclerotic disease of epicardial coronary arteries. By reducing the lumen of these vessels, atherosclerosis causes an absolute decrease in myocardial perfusion in the basal state or limits appropriate increases in perfusion when the demand for flow is augmented. Coronary blood flow can also be limited by arterial thrombi, spasm, and, rarely, coronary emboli, as well as by ostial narrowing due to luetic aortitis. Congenital abnormalities, such as anomalous origin of the left anterior descending coronary artery from the pulmonary artery, may cause myocardial ischemia and infarction in infancy, but this cause is very rare in adults. Myocardial ischemia can also occur if myocardial oxygen demands are abnormally increased, as in severe ventricular hypertrophy due to hypertension or aortic stenosis. The latter can be present with angina that is indistinguishable from that caused by coronary atherosclerosis. A reduction in the oxygen-carrying capacity of the blood, as in extremely severe anemia or in the presence of carboxy-hemoglobin, is a rare cause of myocardial ischemia. Not infrequently, two or more causes of ischemia will coexist, such as an increase in oxygen demand due to left ventricular hypertrophy and a reduction in oxygen supply secondary to coronary atherosclerosis.

The foregoing studies are aimed at defining the role of particular gene variations presumed to be involved in the misleading of normal cellular function leading to cardiovascular disease. However, such approaches cannot identify the full panoply of gene variations that are involved in the disease process.

At present, the only available treatments for cardiovascular disorders are pharmaceutical based medications that are not targeted to an individual's actual defect; examples include angiotensin converting enzyme (ACE) inhibitors and diuretics for hypertension, insulin supplementation for non-insulin dependent diabetes mellitus (NIDDM), cholesterol reduction strategies for dyslipidaemia, anticoagulants, β blockers for cardiovascular disorders and weight reduction strategies for obesity. If targeted treatment strategies were available it might be possible to predict the response to a particular regime of therapy and could markedly increase the effectiveness of such treatment. Although targeted therapy requires accurate diagnostic tests for disease susceptibility, once these tests are developed the opportunity to utilize targeted therapy will become widespread. Such diagnostic tests could initially serve to identify individuals at most risk of hypertension and could allow them to make changes in lifestyle or diet that would serve as preventative measures. The benefits associated by coupling the diagnostic tests with a system of targeted therapy could include the reduction in dosage of administered drugs and thus the amount of unpleasant side effects suffered by an individual. In more severe cases a diagnostic test may suggest that earlier surgical intervention would be useful in preventing a further deterioration in condition.

It is an object of the invention to provide genetic diagnosis of predisposition or susceptibility for cardiovascular diseases. Another related object is to provide treatment to reduce or prevent or delay the onset of disease in those predisposed or susceptible to this disease. A further object is to provide means for carrying out this diagnosis.

Accordingly, a first aspect of the invention provides a method of diagnosis of disease in an individual, said method comprising determining one, various or all genotypes in said individual of the genes listed in the Examples.

In another aspect, the invention provides a method of identifying an individual predisposed or susceptible to a disease, said method comprising determining one, various or all genotypes in said individual of the genes listed in the Examples.

The invention is of advantage in that it enables diagnosis of a disease or of certain disease states via genetic analysis which can yield useable results before onset of disease symptoms, or before onset of severe symptoms. The invention is further of advantage in that it enables diagnosis of predisposition or susceptibility to a disease or of certain disease states via genetic analysis.

The invention may also be of use in confirming or corroborating the results of other diagnostic methods. The diagnosis of the invention may thus suitably be used either as an isolated technique or in combination with other methods and apparatus for diagnosis, in which latter case the invention provides a further test on which a diagnosis may be assessed.

The present invention stems from using allelic association as a method for genotyping individuals; allowing the investigation of the molecular genetic basis for cardiovascular diseases. In a specific embodiment the invention tests for the polymorphisms in the sequences of the listed genes in the Examples. The invention demonstrates a link between this polymorphisms and predispositions to cardiovascular diseases by showing that allele frequencies significantly differ when individuals with "bad" serum lipids are compared to individuals with "good" serum levels. The meaning of "good and bad" serum lipid levels is defined in Table 1 a.

Certain disease states would benefit, that is to say the suffering of the patient may be reduced or prevented or delayed, by administration of treatment or therapy in advance of disease appearance; this can be more reliably carried out if advance diagnosis of predisposition or susceptibility to disease can be diagnosed.

### Pharmacogenomics and adverse drug reactions

Adverse drug reactions (ADRs) remain a major clinical problem. A recent meta-analysis suggested that in the USA in 1994, ADRs were responsible for 100 000 deaths, making them between the fourth and sixth commonest cause of death (Lazarou 1998, J. Am. Med. Assoc. 279:1200). Although these figures have been heavily criticized, they emphasize the importance of ADRs. Indeed, there is good evidence that ADRs account for 5% of all hospital admissions and increase the length of stay in hospital by two days at an increased cost of ~$2500 per patient. ADRs are also one of the commonest causes of drug withdrawal, which has enormous financial implications for the pharmaceutical industry. ADRs, perhaps fortunately, only affect a minority of those taking a particular drug. Although factors that determine susceptibility are unclear in most cases, there is increasing interest in the role of genetic factors. Indeed, the role of inheritable variations in predisposing patients to ADRs has been appreciated since the late 1950s and early 1960s through the discovery of deficiencies in enzymes such as pseudocholinesterase (butyrylcholinesterase) and glucose-6-phosphate dehydrogenase (G6PD). More recently, with the first draft of the human genome just completed, there has been renewed interest in this area with the introduction of terms such as pharmacogenomics and toxicogenomics . Essentially, the aim of pharmacogenomics is to produce personalized medicines, whereby administration of the drug class and dosage is tailored to an individual genotype. Thus, the term pharmacogenomics embraces both efficacy and toxicity.

The 3-hydroxy-3-methylglutaryl coenzyme A (HMG-CoA) reductase inhibitors ("statins") specifically inhibit the enzyme HMG-CoA reductase which catalyzes the rate limiting step in cholesterol biosynthesis. These drugs are effective in reducing the primary and secondary risk of coronary artery disease and coronary events, such as heart attack, in middle-aged and older men and women, in both diabetic and non-diabetic patients, and are often prescribed for patients with hyperlipidemia. Statins used in secondary prevention of coronary artery or heart disease significantly reduce the risk of stroke, total mortality and morbidity and attacks of myocardial ischemia; the use of statins is also associated with improvements in endothelial and fibrinolytic functions and decreased platelet thrombus formation.

The tolerability of these drugs during long term administration is an important issue. Adverse reactions involving skeletal muscle are not uncommon, and sometimes serious adverse reactions involving skeletal muscle such as myopathy and rhabdomyolysis may occur, requiring discontinuation of the drug. In addition an increase in serum creatine kinase (CK) may be a sign of a statin related adverse event. The extend of such adverse events can be read from the extend of the CK level increase (as compared to the upper normal limit [ULN]).

Occasionally arthralgia, alone or in association with myalgia, has been reported. Also an elevation of liver transaminases has been associated with statin administration.

It was shown that the drug response to statin therapy is a class effects, i.e. all known and presumably also all so far undiscovered statins share the same benefical and harmful effects (Ucar, M. et al., Drug Safety 2000, 22:441 ). It follows that the discovery of diagnostic tools to predict the drug response to a single statin will also be of aid to guide therapy with other statins.

The present invention provides diagnostic tests to predict the patient's individual response to statin therapy. Such responses include, but are not limited by the extent of adverse drug reactions, the level of lipid lowering or the drug's influence on disease states. Those diagnostic tests may predict the response to statin therapy either alone or in combination with another diagnostic test or another drug regimen.

### Detailed Description of the Invention

The present invention is based at least in part on the discovery that a specific allele of a polymorphic region of a so called "candidate gene" (as defined below) is associated with CVD or drug response.

For the present invention the following candidate genes were analyzed:
- Genes found to be expressed in cardiac tissue (Hwang et al., Circulation 1997, 96:4146-4203).
- Genes from the following metabolic pathways and their regulatory elements:

### Lipid metabolism

Numerous studies have shown a connection between serum lipid levels and cardiovascular diseases. Candidate genes falling into this group include but are not limited by genes of the cholesterol pathway, apolipoproteins and their modifying factors.

### Coagulation

Ischemic diseases of the heart and in particular myocardial infarction may be caused by a thrombotic occlusion. Genes falling into this group include all genes of the coagulation cascade and their regulatory elements.

### Inflammation

Complications of atherosclerosis are the most common causes of death in Western societies. In broad outline atherosclerosis can be considered to be a form of chronic inflammation resulting from interaction modified lipoproteins, monocyte-derived macrophages,T cells, and the normal cellular elements of the arterial wall. This inflammatory process can ultimately lead to the development of complex lesions, or plaques, that protrude into the arterial lumen. Finally plaque rupture and thrombosis result in the acute clinical complications of myocardial infarction and stroke (Glass et al., Cell 2001, 104:503-516).

It follows that all genes related to inflammatory processes, including but not limited by cytokines, cytokine receptors and cell adhesion molecules are candidate genes for CVD.

### Glucose and energy metabolism

As glucose and energy metabolism is interdependent with the metabolism of lipids (see above) also the former pathways contain candidate genes. Energy metabolism in general also relates to obesity, which is an independent risk factor for CVD (Melanson et al., Cardiol Rev 2001 9:202-207). In addition high blood glucose levels are associated with many microvascular and macrovascular complications and may therefore affect an individuals disposition to CVD (Duckworth, Curr Atheroscler Rep 2001, 3:383-391).

### Hypertension

As hypertension is an independent risk factor for CVD, also genes that are involved in the regulation of systolic and diastolic blood pressure affect an individuals risk for CVD (Safar, Curr Opin Cardiol 2000, 15:258-263). Interestingly hypertension and diabetes (see above) appear to be interdependent, since hypertension is approximately twice as frequent in patients with diabetes compared with patients without the disease. Conversely, recent data suggest that hypertensive persons are more predisposed to the development of diabetes than are normotensive persons (Sowers et al., Hypertension 2001, 37:1053-1059).

### Genes related to drug response

Those genes include metabolic pathways involved in the absorption, distribution, metabolism, excretion and toxicity (ADMET) of drugs. Prominent members of this group are the cytochrome P450 proteins which catalyze many reactions involved in drug metabolism.

### Unclassified genes

As stated above, the mechanisms that lead to cardiovascular diseases or define the patient's individual response to drugs are not completely elucidated. Hence also candidate genes were analysed, which could not be assigned to the above listed categories. The present invention is based at least in part on the discovery of polymorphisms, that lie in genomic regions of unknown physiological function.

### Results

After conducting an association study, we surprisingly found polymorphic sites in a number of candidate genes which show a strong correlation with the following phenotypes of the patients analysed: "Healthy" as used herein refers to individuals that neither suffer from existing CVD, nor exhibit an increased risk for CVD through their serum lipid level profile. "CVD prone" as used herein refers to individuals with existing CVD and/or a serum lipid profile that confers a high risk to get CVD (see Table 1a for definitions of healthy and CVD prone serum lipid levels). "High responder" as used herein refers to patients who benefit from relatively small amounts of a given drug. "Low responder" as used herein refers to patients who need relatively high doses in order to obtain benefit from the medication. "Tolerant patient" refers to individuals who can tolerate high doses of a medicament without exhibiting adverse drug reactions. "ADR patient" as used herein refers to individuals who suffer from ADR or show clinical symptoms (like creatine kinase elevation in blood) even after receiving only minor doses of a medicament (see Table 1 b for a detailed definition of drug response phenotypes).

Polymorphic sites in candidate genes that were found to be significantly associated with either of the above mentioned phenotypes will be referred to as "phenotype associated SNPs" (PA SNPs). The respective genomic loci that harbour PA SNPs will be referred to as "phenotype associated genes" (PA genes), irrespective of the actual function of this gene locus.

In particular we surprisingly found PA SNPs associated with CVD, drug efficacy (EFF) or adverse drug reactions (ADR) in the following genes.

### ABCA1: ATP-binding cassette, sub-family A (ABC1), member 1

The membrane-associated protein encoded by this gene is a member of the superfamily of ATP-binding cassette (ABC) transporters. ABC proteins transport various molecules across extra- and intracellular membranes. ABC genes are divided into seven distinct subfamilies (ABC1, MDR/TAP, MRP, ALD, OABP, GCN20, White). This protein is a member of the ABC1 subfamily. Members of the ABC1 subfamily comprise the only major ABC subfamily found exclusively in multicellular eukaryotes. With cholesterol as its substrate, this protein functions as a cholesteral efflux pump in the cellular lipid removal pathway. Mutations in this gene have been associated with Tangier's disease and familial high-density lipoprotein deficiency.

### ABCB1: ATP-binding cassette, sub-family B (MDR/TAP), member 1

The membrane-associated protein encoded by this gene is a member of the superfamily of ATP-binding cassette (ABC) transporters. ABC proteins transport various molecules across extra- and intra-cellular membranes. ABC genes are divided into seven distinct subfamilies (ABC1, MDR/TAP, MRP, ALD, OABP, GCN20, White). This protein is a member of the MDR/TAP subfamily. Members of the MDR/TAP subfamily are involved in multidrug resistance. The protein encoded by this gene is an ATP-dependent drug efflux pump for xenobiotic compounds with broad substrate specificity. It is responsible for decreased drug accumulation in multidrug-resistant cells and often mediates the development of resistance to anticancer drugs. This protein also functions as a transporter in the blood-brain barrier.

### ABCG2: ATP-binding cassette, sub-family G (WHITE), member 2

The membrane-associated protein encoded by this gene is included in the superfamily of ATP-binding cassette (ABC) transporters. ABC proteins transport various molecules across extra- and intra-cellular membranes. ABC genes are divided into seven distinct subfamilies (ABC1, MDR/TAP, MRP, ALD, OABP, GCN20, White). This protein is a member of the White subfamily. Alternatively referred to as a breast cancer resistance protein, this protein functions as a xenobiotic transporter which may play a major role in multi-drug resistance. It likely serves as a cellular defense mechanism in response to mitoxantrone and anthracycline exposure. Significant expression of this protein has been observed in the placenta, which may suggest a potential role for this molecule in placenta tissue.

### CD69: CD69 antigen (p60, early T-cell activation antigen)

Member of the Ca2+-dependent (C-type) lectin superfamily of type II transmembrane receptors; acts as signal transducing receptor, involved in lymphocyte proliferation.

### CSF3: colony stimulating factor 3 (granulocyte)

Granulocyte colony stimulating factor 3.

### Hexokinase II

Hexokinases phosphorylate glucose to produce glucose-6-phosphate, thus committing glucose to the glycolytic pathway. This gene encodes hexokinase 2, the predominant form found in skeletal muscle. It localizes to the outer membrane of mitochondria. Expression of this gene is insulin-responsive, and studies in rat suggest that it is involved in the increased rate of glycolysis seen in rapidly growing cancer cells.

### Human bcl-2 mRNA.

BCL2 is an integral inner mitochondrial membrane protein that blocks the apoptotic death of some cells such as lymphocytes. Constitutive expression of BCL2, such as in the case of translocation of BCL2 to Ig heavy chain locus, is thought to be the cause of follicular lymphoma. Two transcript variants, produced by alternate splicing, differ in their C-terminal ends. Variant 1 is represented by a 5.5 kb mRNA and includes most of exon 1 and exon 2. A 3.5 kb mRNA represents variant 2; it includes all of exon 1 and lacks exon 2 sequence.

### Human methylenetetrahydrofolate dehydrogenase- methenyltetrahydrofolate cyclohydrolase-formyltetrahydrofolate synthetase mRNA, complete cds.

This gene encodes a protein that possesses three distinct enzymatic activities, 5,10-methylenetetrahydrofolate dehydrogenase, 5,10-methenyltetrahydrofolate cyclohydrolase and 10-formyltetrahydrofolate synthetase. Each of these activities catalyzes one of three sequential reactions in the interconversion of 1-carbon derivatives of tetrahydrofolate, which are substrates for methionine, thymidylate, and de novo purine syntheses. The trifunctional enzymatic activities are conferred by two major domains, an aminoterminal portion containing the dehydrogenase and cyclohydrolase activities and a larger synthetase domain.

### Human thermostable phenol sulfotransferase (STP2) gene

Phenol-metabolizing sulfotransferase 2; sulfonates simple planar phenols.

### IFIT2: interferon-induced protein with tetratricopeptide repeats 2 (ISG-54K)

### IL4: interleukin 4

Interleukin 4; cytokine that stimulates the proliferation of T cells.

### ITGA9: integrin, alpha 9

Alpha 9 subunit of integrin; may be involved in cell-cell and cell-matrix interactions; member of a family of cell-surface proteins.

### KIAA0229: KIAA0229 protein

### MMP1: matrix metalloproteinase 1 (interstitial collagenase)

Proteins of the matrix metalloproteinase (MMP) family are involved in the breakdown of extracellular matrix in normal physiological processes, such as embryonic development, reproduction, and tissue remodeling, as well as in disease processes, such as arthritis and metastasis. Most MMP's are secreted as inactive proproteins which are activated when cleaved by extracellular proteinases. This gene encodes a secreted enzyme which breaks down the interstitial collagens, types I, II, and III. The gene is part of a cluster of MMP genes which localize to chromosome 11q22.3.

### MMP16: matrix metalloproteinase 16 (membrane-inserted)

Proteins of the matrix metalloproteinase (MMP) family are involved in the breakdown of extracellular matrix in normal physiological processes, such as embryonic development, reproduction, and tissue remodeling, as well as in disease processes, such as arthritis and metastasis. Most MMP's are secreted as inactive proproteins which are activated when cleaved by extracellular proteinases. This gene produces two transcripts, which encode a membrane-bound form and a soluble form of the protein. Both forms of the protein activate MMP2 by cleavage. This gene was once referred to as MT-MMP2, but was renamed as MT-MMP3 or MMP16.

### OXA1Hs

Involved in the assembly of complexes of the electron transport chain.

### PROCR: protein C receptor, endothelial (EPCR)

Endothelial Protein C receptor; binds protein C in a calcium-dependent manner; member of the CD1/major histocompatibility complex superfamily.

### SERPINE1: serine (or cysteine) proteinase inhibitor, clade E (nexin, plasminogen activator inhibitor type 1), member 1

Plasminogen activator inhibitor I; regulates fibrinolysis; member of the serpin family of serine protease inhibitors.

### STX1A: syntaxin 1A (brain)

Syntaxin 1A (brain); involved in intracellular transport and neurotransmitter release.

As SNPs are linked to other SNPs in neighboring genes on a chromosome (Linkage Disequilibrium) those SNPs could also be used as marker SNPs. In a recent publication it was shown that SNPs are linked over 100 kb in some cases more than 150 kb (Reich D.E. et al. Nature 411, 199-204, 2001). Hence SNPs lying in regions neighbouring PA SNPs could be linked to the latter and by this being a diagnostic marker. These associations could be performed as described for the gene polymorphism in methods.

### Definitions

For convenience, the meaning of certain terms and phrases employed in the specification, examples, and appended claims are provided below. Moreover, the definitions by itself are intended to explain a further background of the invention.

The term "allele", which is used interchangeably herein with "allelic variant" refers to alternative forms of a gene or portions thereof. Alleles occupy the same locus or position on homologous chromosomes. When a subject has two identical alleles of a gene, the subject is said to be homozygous for the gene or allele. When a subject has two different alleles of a gene, the subject is said to be heterozygous for the gene. Alleles of a specific gene can differ from each other in a single nucleotide, or several nucleotides, and can include substitutions, deletions, and insertions of nucleotides. An allele of a gene can also be a form of a gene containing a mutation.

The term "allelic variant of a polymorphic region of a gene" refers to a region of a gene having one of several nucleotide sequences found in that region of the gene in other individuals.

"Homology" or "identity" or "similarity" refers to sequence similarity between two peptides or between two nucleic acid molecules. Homology can be determined by comparing a position in each sequence which may be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares less than 40% identity, though preferably less than 25% identity, with one of the sequences of the present invention.

The term "a homologue of a nucleic acid" refers to a nucleic acid having a nucleotide sequence having a certain degree of homology with the nucleotide sequence of the nucleic acid or complement thereof. A homologue of a double stranded nucleic acid having SEQ ID NO. X is intended to include nucleic acids having a nucleotide sequence which has a certain degree of homology with SEQ ID NO. X or with the complement thereof. Preferred homologous of nucleic acids are capable of hybridizing to the nucleic acid or complement thereof.

The term "interact" as used herein is meant to include detectable interactions between molecules, such as can be detected using, for example, a hybridization assay.

The term interact is also meant to include "binding" interactions between molecules. Interactions may be, for example, protein-protein, protein-nucleic acid, protein-small molecule or small molecule-nucleic acid in nature.

The term "intronic sequence" or "intronic nucleotide sequence" refers to the nucleotide sequence of an intron or portion thereof.

The term "isolated" as used herein with respect to nucleic acids, such as DNA or RNA, refers to molecules separated from other DNAs or RNAs, respectively, that are present in the natural source of the macromolecule. The term isolated as used herein also refers to a nucleic acid or peptide that is substantially free of cellular material, viral material, or culture medium when produced by recombinant DNA techniques, or chemical precursors or other chemicals when chemically synthesized.

Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides.

The term "lipid" shall refer to a fat or fat-like substance that is insoluble in polar solvents such as water. The term "lipid" is intended to include true fats (e.g. esters of fatty acids and glycerol); lipids (phospholipids, cerebrosides, waxes); sterols (cholesterol, ergosterol) and lipoproteins (e.g. HDL, LDL and VLDL).

The term "locus" refers to a specific position in a chromosome. For example, a locus of a gene refers to the chromosomal position of the gene.

The term "modulation" as used herein refers to both up-regulation, (i.e., activation or stimulation), for example by agonizing, and down-regulation (i.e. inhibition or suppression), for example by antagonizing of a bioactivity (e.g. expression of a gene).

The term "molecular structure" of a gene or a portion thereof refers to the structure as defined by the nucleotide content (including deletions, substitutions, additions of one or more nucleotides), the nucleotide sequence, the state of methylation, and/or any other modification of the gene or portion thereof.

The term "mutated gene" refers to an allelic form of a gene, which is capable of altering the phenotype of a subject having the mutated gene relative to a subject which does not have the mutated gene. If a subject must be homozygous for this mutation to have an altered phenotype, the mutation is said to be recessive. If one copy of the mutated gene is sufficient to alter the genotype of the subject, the mutation is said to be dominant. If a subject has one copy of the mutated gene and has a phenotype that is intermediate between that of a homozygous and that of a heterozygous (for that gene) subj ect, the mutation is said to be co-dominant.

As used herein, the term "nucleic acid" refers to polynucleotides such as deoxyribonucleic acid (DNA), and, where appropriate, ribonucleic acid (RNA). The term should also be understood to include, as equivalents, derivatives, variants and analogs of either RNA or DNA made from nucleotide analogs, including peptide nucleic acids (PNA), morpholino oligonucleotides (J. Summerton and D. Weller, Antisense and Nucleic Acid Drug Development 7:187 (1997)) and, as applicable to the embodiment being described, single (sense or antisense) and double-stranded polynucleotides. Deoxyribonucleotides include deoxyadenosine, deoxycytidine, deoxyguanosine, and deoxythymidine. For purposes of clarity, when referring herein to a nucleotide of a nucleic acid, which can be DNA or an RNA, the term "adenosine", "cytidine", "guanosine", and "thymidine" are used. It is understood that if the nucleic acid is RNA, a nucleotide having a uracil base is uridine.

The term "nucleotide sequence complementary to the nucleotide sequence set forth in SEQ ID NO. x" refers to the nucleotide sequence of the complementary strand of a nucleic acid strand having SEQ ID NO. x. The term "complementary strand" is used herein interchangeably with the term "complement". The complement of a nucleic acid strand can be the complement of a coding strand or the complement of a non-coding strand. When referring to double stranded nucleic acids, the complement of a nucleic acid having SEQ ID NO. x refers to the complementary strand of the strand having SEQ ID NO. x or to any nucleic acid having the nucleotide sequence of the complementary strand of SEQ ID NO. x. When referring to a single stranded nucleic acid having the nucleotide sequence SEQ ID NO. x, the complement of this nucleic acid is a nucleic acid having a nucleotide sequence which is complementary to that of SEQ ID NO. x. The nucleotide sequences and complementary sequences thereof are always given in the 5' to 3' direction. The term "complement" and "reverse complement" are used interchangeably herein.

The term "operably linked" is intended to mean that the promoter is associated with the nucleic acid in such a manner as to facilitate transcription of the nucleic acid.

The term "polymorphism" refers to the coexistence of more than one form of a gene or portion thereof. A portion of a gene of which there are at least two different forms, i.e., two different nucleotide sequences, is referred to as a "polymorphic region of a gene". A polymorphic region can be a single nucleotide, the identity of which differs in different alleles. A polymorphic region can also be several nucleotides long.

A "polymorphic gene" refers to a gene having at least one polymorphic region.

To describe a "polymorphic site" in a nucleotide sequence often there is used an "ambiguity code" that stands for the possible variations of nucleotides in one site. The list of ambiguity codes is summarized in the following table:

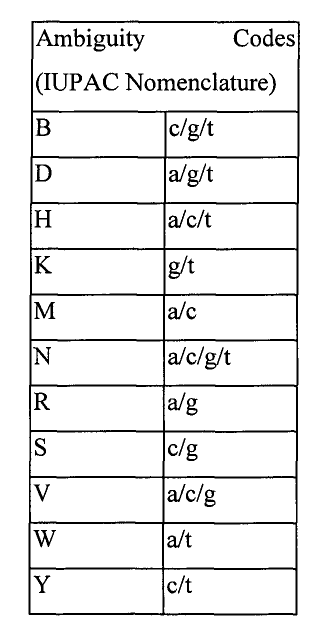

So, for example, a "R" in a nucleotide sequence means that either an "a" or a "g" could be at that position.

The terms "protein", "polypeptide" and "peptide" are used interchangeably herein when referring to a gene product.

A "regulatory element", also termed herein "regulatory sequence is intended to include elements which are capable of modulating transcription from a basic promoter and include elements such as enhancers and silencers. The term "enhancer", also referred to herein as "enhancer element", is intended to include regulatory elements capable of increasing, stimulating, or enhancing transcription from a basic promoter. The term "silencer", also referred to herein as "silencer element" is intended to include regulatory elements capable of decreasing, inhibiting, or repressing transcription from a basic promoter. Regulatory elements are typically present in 5' flanking regions of genes. However, regulatory elements have also been shown to be present in other regions of a gene, in particular in introns. Thus, it is possible that genes have regulatory elements located in introns, exons, coding regions, and 3' flanking sequences. Such regulatory elements are also intended to be encompassed by the present invention and can be identified by any of the assays that can be used to identify regulatory elements in 5' flanking regions of genes.

The term "regulatory element" further encompasses "tissue specific" regulatory elements, i.e., regulatory elements which effect expression of the selected DNA sequence preferentially in specific cells (e.g., cells of a specific tissue). gene expression occurs preferentially in a specific cell if expression in this cell type is significantly higher than expression in other cell types. The term "regulatory element" also encompasses non-tissue specific regulatory elements, i.e., regulatory elements which are active in most cell types. Furthermore, a regulatory element can be a constitutive regulatory element, i.e., a regulatory element which constitutively regulates transcription, as opposed to a regulatory element which is inducible, i.e., a regulatory element which is active primarily in response to a stimulus. A stimulus can be, e.g., a molecule, such as a hormone, cytokine, heavy metal, phorbol ester, cyclic AMP (cAMP), or retinoic acid.

Regulatory elements are typically bound by proteins, e.g., transcription factors. The term "transcription factor" is intended to include proteins or modified forms thereof, which interact preferentially with specific nucleic acid sequences, i.e., regulatory elements, and which in appropriate conditions stimulate or repress transcription. Some transcription factors are active when they are in the form of a monomer. Alternatively, other transcription factors are active in the form of a dimer consisting of two identical proteins or different proteins (heterodimer). Modified forms of transcription factors are intended to refer to transcription factors having a post-translational modification, such as the attachment of a phosphate group. The activity of a transcription factor is frequently modulated by a post-translational modification. For example, certain transcription factors are active only if they are phosphorylated on specific residues. Alternatively, transcription factors can be active in the absence of phosphorylated residues and become inactivated by phosphorylation. A list of known transcription factors and their DNA binding site can be found, e.g., in public databases, e.g., TFMATRIX Transcription Factor Binding Site Profile database.

As used herein, the term "specifically hybridizes" or "specifically detects" refers to the ability of a nucleic acid molecule of the invention to hybridize to at least approximately 6, 12, 20, 30, 40, 50, 60, 70, 80, 90, 100, 110, 120, 130 or 140 consecutive nucleotides of either strand of a gene.

The term "wild-type allele" refers to an allele of a gene which, when present in two copies in a subject results in a wild-type phenotype. There can be several different wild-type alleles of a specific gene, since certain nucleotide changes in a gene may not affect the phenotype of a subject having two copies of the gene with the nucleotide changes.

"Adverse drug reaction" (ADR) as used herein refers to an appreciably harmful or unpleasant reaction, resulting from an intervention related to the use of a medicinal product, which predicts hazard from future administration and warrants prevention or specific treatment, or alteration of the dosage regimen, or withdrawal of the product. In it's most severe form an ADR might lead to the death of an individual.

The term "Drug Response" is intended to mean any response that a patient exhibits upon drug administration. Specifically drug response includes beneficial, i.e. desired drug effects, ADR or no detectable reaction at all. More specifically the term drug response could also have a qualitative meaning, i.e. it embraces low or high beneficial effects, respectively and mild or severe ADR, respectively. The term "Statin Response" as used herein refers to drug response after statin administration. An individual drug response includes also a good or bad metabolizing of the drug, meaning that "bad metabolizers" accumulate the drug in the body and by this could show side effects of the drug due to accumulative overdoses.

"Candidate gene" as used herein includes genes that can be assigned to either normal cardiovascular function or to metabolic pathways that are related to onset and/or progression of cardiovascular diseases.

With regard to drug response the term "candidate gene" includes genes that can be assigned to distinct phenotypes regarding the patient's response to drug administration. Those phenotypes may include patients who benefit from relatively small amounts of a given drug (high responders) or patients who need relatively high doses in order to obtain the same benefit (low responders). In addition those phenotypes may include patients who can tolerate high doses of a medicament without exhibiting ADR, or patients who suffer from ADR even after receiving only low doses of a medicament.

As neither the development of cardiovascular diseases nor the patient's response to drug administration is completely understood, the term "candidate gene" may also comprise genes with presently unknown function.

"PA SNP" (phenotype associated SNP) refers to a polymorphic site which shows a significant association with a patients phenotype (healthy, diseased, low or high responder, drug tolerant, ADR prone, etc.)

"PA gene" (phenotype associated gene) refers to a genomic locus harbouring a PA SNP, irrespective of the actual function of this gene locus.

PA gene polypeptide refers to a polypeptide encoded at least in part by a PA gene.

The term "Haplotype" as used herein refers to a group of two or more SNPs that are functionally and/or spatially linked. I.e. haplotypes define groups of SNPs that lie inside genes belonging to identical (or related metabolic) pathways and/or lie on the same chromosome. Haplotypes are expected to give better predictive/diagnostic information than a single SNP

The term "statin" is intended to embrace all inhibitors of the enzyme 3-hydroxy-3-methylglutaryl coenzyme A (HMG-CoA) reductase. Statins specifically inhibit the enzyme HMG-CoA reductase which catalyzes the rate limiting step in cholesterol biosynthesis. Known statins are Atorvastatin, Cerivastatin, Fluvastatin, Lovastatin, Pravastatin and Simvastatin.

### Methods for Assessing Cardiovascular Status

The present invention provides diagnostic methods for assessing cardiovascular status in a human individual. Cardiovascular status as used herein refers to the physiological status of an individual's cardiovascular system as reflected in one or more markers or indicators. Status markers include without limitation clinical measurements such as, e.g., blood pressure, electrocardiographic profile, and differentiated blood flow analysis as well as measurements of LDL- and HDL-Cholesterol levels, other lipids and other well established clinical parameters that are standard in the art. Status markers according to the invention include diagnoses of one or more cardiovascular syndromes, such as, e.g., hypertension, acute myocardial infarction, silent myocardial infarction, stroke, and atherosclerosis. It will be understood that a diagnosis of a cardiovascular syndrome made by a medical practitioner encompasses clinical measurements and medical judgement. Status markers according to the invention are assessed using conventional methods well known in the art. Also included in the evaluation of cardiovascular status are quantitative or qualitative changes in status markers with time, such as would be used, e.g., in the determination of an individual's response to a particular therapeutic regimen.

The methods are carried out by the steps of:
(i) determining the sequence of one or more polymorphic positions within one, several or all of the genes listed in Examples or other genes mentioned in this file in the individual to establish a polymorphic pattern for the individual; and
(ii) comparing the polymorphic pattern established in (i) with the polymorphic patterns of humans exhibiting different markers of cardiovascular status. The polymorphic pattern of the individual is, preferably, highly similar and, most preferably, identical to the polymorphic pattern of individuals who exhibit particular status markers, cardiovascular syndromes, and/or particular patterns of response to therapeutic interventions. Polymorphic patterns may also include polymorphic positions in other genes which are shown, in combination with one or more polymorphic positions in the genes listed in the Examples, to correlate with the presence of particular status markers. In one embodiment, the method involves comparing an individual's polymorphic pattern with polymorphic patterns of individuals who have been shown to respond positively or negatively to a particular therapeutic regimen. Therapeutic regimen as used herein refers to treatments aimed at the elimination or amelioration of symptoms and events associated cardiovascular disease. Such treatments include without limitation one or more of alteration in diet, lifestyle, and exercise regimen; invasive and noninvasive surgical techniques such as atherectomy, angioplasty, and coronary bypass surgery; and pharmaceutical interventions, such as administration of ACE inhibitors, angiotensin II receptor antagonists, diuretics, alpha-adrenoreceptor antagonists, cardiac glycosides, phosphodiesterase inhibitors, beta-adrenoreceptor antagonists, calcium channel blockers, HMG-CoA reductase inhibitors, imidazoline receptor blockers, endothelin receptor blockers, organic nitrites, and modulators of protein function of genes listed in the Examples. Interventions with pharmaceutical agents not yet known whose activity correlates with particular polymorphic patterns associated with cardiovascular disease are also encompassed. It is contemplated, for example, that patients who are candidates for a particular therapeutic regimen will be screened for polymorphic patterns that correlate with responsivity to that particular regimen.

In a preferred embodiment, the method involves comparing an individual's polymorphic pattern with polymorphic patterns of individuals who exhibit or have exhibited one or more markers of cardiovascular disease, such as, e.g., elevated LDL-Cholesterol levels, high blood pressure, abnormal electrocardiographic profile, myocardial infarction, stroke, or atherosclerosis.

In another embodiement, the method involves comparing an individual's polymorphic pattern with polymorphic patterns of individuals who exhibit or have exhibited one or more drug related phenotypes, such as, e.g., low or high drug response, or adverse drug reactions.

In practicing the methods of the invention, an individual's polymorphic pattern can be established by obtaining DNA from the individual and determining the sequence at predetermined polymorphic positions in the genes such as those described in this file.

The DNA may be obtained from any cell source. Non-limiting examples of cell sources available in clinical practice include blood cells, buccal cells, cervicovaginal cells, epithelial cells from urine, fetal cells, or any cells present in tissue obtained by biopsy. Cells may also be obtained from body fluids, including without limitation blood, saliva, sweat, urine, cerebrospinal fluid, feces, and tissue exudates at the site of infection or inflammation. DNA is extracted from the cell source or body fluid using any of the numerous methods that are standard in the art. It will be understood that the particular method used to extract DNA will depend on the nature of the source.

### Diagnostic and Prognostic Assays

The present invention provides methods for determining the molecular structure of at least one polymorphic region of a gene, specific allelic variants of said polymorphic region being associated with cardiovascular disease. In one embodiment, determining the molecular structure of a polymorphic region of a gene comprises determining the identity of the allelic variant. A polymorphic region of a gene, of which specific alleles are associated with cardiovascular disease can be located in an exon, an intron, at an intron/exon border, or in the promoter of the gene.

The invention provides methods for determining whether a subject has, or is at risk, of developing a cardiovascular disease. Such disorders can be associated with an aberrant gene activity, e.g., abnormal binding to a form of a lipid, or an aberrant gene protein level. An aberrant gene protein level can result from an aberrant transcription or post-transcriptional regulation. Thus, allelic differences in specific regions of a gene can result in differences of gene protein due to differences in regulation of expression. In particular, some of the identified polymorphisms in the human gene may be associated with differences in the level of transcription, RNA maturation, splicing, or translation of the gene or transcription product.

In preferred embodiments, the methods of the invention can be characterized as comprising detecting, in a sample of cells from the subject, the presence or absence of a specific allelic variant of one or more polymorphic regions of a gene. The allelic differences can be: (i) a difference in the identity of at least one nucleotide or (ii) a difference in the number of nucleotides, which difference can be a single nucleotide or several nucleotides.

A preferred detection method is allele specific hybridization using probes overlapping the polymorphic site and having about 5, 10, 20, 25, or 30 nucleotides around the polymorphic region. Examples of probes for detecting specific allelic variants of the polymorphic region located in intron X are probes comprising a nucleotide sequence set forth in any of SEQ ID NO. X. In a preferred embodiment of the invention, several probes capable of hybridizing specifically to allelic variants are attached to a solid phase support, e.g., a "chip". Oligonucleotides can be bound to a solid support by a variety of processes, including lithography. For example a chip can hold up to 250,000 oligonucleotides (GeneChip, Affymetrix). Mutation detection analysis using these chips comprising oligonucleotides, also termed "DNA probe arrays" is described e.g., in Cronin et al. (1996) Human Mutation 7:244 and in Kozal et al. (1996) Nature Medicine 2:753. In one embodiment, a chip comprises all the allelic variants of at least one polymorphic region of a gene. The solid phase support is then contacted with a test nucleic acid and hybridization to the specific probes is detected. Accordingly, the identity of numerous allelic variants of one or more genes can be identified in a simple hybridization experiment. For example, the identity of the allelic variant of the nucleotide polymorphism of nucleotide A or G at position 33 of Seq ID 1 (baySNP179) and that of other possible polymorphic regions can be determined in a single hybridization experiment.

In other detection methods, it is necessary to first amplify at least a portion of a gene prior to identifying the allelic variant. Amplification can be performed, e.g., by PCR and/or LCR, according to methods known in the art. In one embodiment, genomic DNA of a cell is exposed to two PCR primers and amplification for a number of cycles sufficient to produce the required amount of amplified DNA. In preferred embodiments, the primers are located between 40 and 350 base pairs apart. Preferred primers for amplifying gene fragments of genes of this file are listed in Table 2 in the Examples.

Alternative amplification methods include: self sustained sequence replication (Guatelli, J. C. et al., 1990, Proc. Natl. Acad. Sci. U.S.A. 87:1874-1878), transcriptional amplification system (Kwoh, D. Y. et al., 1989, Proc. Natl. Acad. Sci. U.S.A. 86:1173-1177), Q-Beta Replicase (Lizardi, P. M. et al., 1988, Bio/Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In one embodiment, any of a variety of sequencing reactions known in the art can be used to directly sequence at least a portion of a gene and detect allelic variants, e.g., mutations, by comparing the sequence of the sample sequence with the corresponding wild-type (control) sequence. Exemplary sequencing reactions include those based on techniques developed by Maxam and Gilbert (Proc. Natl Acad Sci USA (1977) 74:560) or Sanger (Sanger et al (1977) Proc. Nat. Acad. Sci 74:5463). It is also contemplated that any of a variety of automated sequencing procedures may be utilized when performing the subject assays (Biotechniques (1995) 19:448), including sequencing by mass spectrometry (see, for example, U.S. Pat. No. 5,547,835 and international patent application Publication Number WO 94/16101, entitled DNA Sequencing by Mass Spectrometry by H. Koster; U.S. Pat. No. 5,547,835 and international patent application Publication Number WO 94/21822 entitled "DNA Sequencing by Mass Spectrometry Via Exonuclease Degradation" by H. Koster), and U.S. Pat. No. 5,605,798 and International Patent Application No. PCT/US96/03651 entitled DNA Diagnostics Based on Mass Spectrometry by H. Koster; Cohen et al. (1996) Adv Chromatogr 36:127-162; and Griffin et al. (1993) Appl Biochem Biotechnol 38:147-159). It will be evident to one skilled in the art that, for certain embodiments, the occurrence of only one, two or three of the nucleic acid bases need be determined in the sequencing reaction. For instance, A-track or the like, e.g., where only one nucleotide is detected, can be carried out.

Yet other sequencing methods are disclosed, e.g., in U.S. Pat. No. 5,580,732 entitled "Method of DNA sequencing employing a mixed DNA-polymer chain probe" and U.S. Pat. No. 5,571,676 entitled "Method for mismatch-directed in vitro DNA sequencing".

In some cases, the presence of a specific allele of a gene in DNA from a subject can be shown by restriction enzyme analysis. For example, a specific nucleotide polymorphism can result in a nucleotide sequence comprising a restriction site which is absent from the nucleotide sequence of another allelic variant.

In other embodiments, alterations in electrophoretic mobility is used to identify the type of gene allelic variant. For example, single strand conformation polymorphism (SSCP) may be used to detect differences in electrophoretic mobility between mutant and wild type nucleic acids (Orita et al. (1989) Proc Natl. Acad. Sci USA 86:2766, see also Cotton (1993) Mutat Res 285:125-144; and Hayashi (1992) Genet Anal Tech Appl 9:73-79). Single-stranded DNA fragments of sample and control nucleic acids are denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In another preferred embodiment, the subject method utilizes heteroduplex analysis to separate double stranded heteroduplex molecules on the basis of changes in electrophoretic mobility (Keen et al. (1991) Trends Genet 7:5).

In yet another embodiment, the identity of an allelic variant of a polymorphic region is obtained by analyzing the movement of a nucleic acid comprising the polymorphic region in polyacrylamide gels containing a gradient of denaturant is assayed using denaturing gradient gel electrophoresis (DGGE) (Myers et al (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing agent gradient to identify differences in the mobility of control and sample DNA (Rosenbaum and Reissner (1987) Biophys Chem 265:1275).

Examples of techniques for detecting differences of at least one nucleotide between 2 nucleic acids include, but are not limited to, selective oligonucleotide hybridization, selective amplification, or selective primer extension. For example, oligonucleotide probes may be prepared in which the known polymorphic nucleotide is placed centrally (allele-specific probes) and then hybridized to target DNA under conditions which permit hybridization only if a perfect match is found (Saiki et al. (1986) Nature 324:163); Saiki et al (1989) Proc. Natl Acad. Sci USA 86:6230; and Wallace et al. (1979) Nucl. Acids Res. 6:3543). Such allele specific oligonucleotide hybridization techniques may be used for the simultaneous detection of several nucleotide changes in different polymorphic regions of gene. For example, oligonucleotides having nucleotide sequences of specific allelic variants are attached to a hybridizing membrane and this membrane is then hybridized with labeled sample nucleic acid. Analysis of the hybridization signal will then reveal the identity of the nucleotides of the sample nucleic acid.

Alternatively, allele specific amplification technology which depends on selective PCR amplification may be used. Oligonucleotides used as primers for specific amplification may carry the allelic variant of interest in the center of the molecule (so that amplification depends on differential hybridization) (Gibbs et al (1989) Nucleic Acids Res. 17:2437-2448) or at the extreme 3' end of one primer where, under appropriate conditions, mismatch can prevent, or reduce polymerase extension (Prossner (1993) Tibtech 11:238; Newton et al. (1989) Nucl. Acids Res. 17:2503). This technique is also termed "PROBE" for Probe Oligo Base Extension. In addition it may be desirable to introduce a novel restriction site in the region of the mutation to create cleavage-based detection (Gasparini et al (1992) Mol. Cell Probes 6:1).

In another embodiment, identification of the allelic variant is carried out using an oligonucleotide ligation assay (OLA), as described, e.g., in U.S. Pat. No. 4,998,617 and in Landegren, U. et al., Science 241:1077-1080 (1988). The OLA protocol uses two oligonucleotides which are designed to be capable of hybridizing to abutting sequences of a single strand of a target. One of the oligonucleotides is linked to a separation marker, e.g,. biotinylated, and the other is detectably labeled. If the precise complementary sequence is found in a target molecule, the oligonucleotides will hybridize such that their termini abut, and create a ligation substrate. Ligation then permits the labeled oligonucleotide to be recovered using avidin, or another biotin ligand. Nickerson, D. A. et al. have described a nucleic acid detection assay that combines attributes of PCR and OLA (Nickerson, D. A. et al., Proc. Natl. Acad. Sci. (U.S.A.) 87:8923-8927 (1990). In this method, PCR is used to achieve the exponential amplification of target DNA, which is then detected using OLA.

Several techniques based on this OLA method have been developed and can be used to detect specific allelic variants of a polymorphic region of a gene. For example, U.S. Pat. No. 5,593,826 discloses an OLA using an oligonucleotide having 3'-amino group and a 5'-phosphorylated oligonucleotide to form a conjugate having a phosphoramidate linkage. In another variation of OLA described in Tobe et al. ((1996)Nucleic Acids Res 24: 3728), OLA combined with PCR permits typing of two alleles in a single microtiter well. By marking each of the allele-specific primers with a unique hapten, i.e. digoxigenin and fluorescein, each LA reaction can be detected by using hapten specific antibodies that are labeled with different enzyme reporters, alkaline phosphatase or horseradish peroxidase. This system permits the detection of the two alleles using a high throughput format that leads to the production of two different colors.

The invention further provides methods for detecting single nucleotide polymorphisms in a gene. Because single nucleotide polymorphisms constitute sites of variation flanked by regions of invariant sequence, their analysis requires no more than the determination of the identity of the single nucleotide present at the site of variation and it is unnecessary to determine a complete gene sequence for each patient. Several methods have been developed to facilitate the analysis of such single nucleotide polymorphisms.

In one embodiment, the single base polymorphism can be detected by using a specialized exonuclease-resistant nucleotide, as disclosed, e.g., in Mundy, C. R. (U.S. Pat. No. 4,656,127). According to the method, a primer complementary to the allelic sequence immediately 3' to the polymorphic site is permitted to hybridize to a target molecule obtained from a particular animal or human. If the polymorphic site on the target molecule contains a nucleotide that is complementary to the particular exonuclease-resistant nucleotide derivative present, then that derivative will be incorporated onto the end of the hybridized primer. Such incorporation renders the primer resistant to exonuclease, and thereby permits its detection. Since the identity of the exonuclease-resistant derivative of the sample is known, a finding that the primer has become resistant to exonucleases reveals that the nucleotide present in the polymorphic site of the target molecule was complementary to that of the nucleotide derivative used in the reaction. This method has the advantage that it does not require the determination of large amounts of extraneous sequence data.

In another embodiment of the invention, a solution-based method is used for determining the identity of the nucleotide of a polymorphic site. Cohen, D. et al. (French Patent 2,650,840; PCT Appln. No. WO91/02087). As in the Mundy method of U.S. Pat. No. 4,656,127, a primer is employed that is complementary to allelic sequences immediately 3' to a polymorphic site. The method determines the identity of the nucleotide of that site using labeled dideoxynucleotide derivatives, which, if complementary to the nucleotide of the polymorphic site will become incorporated onto the terminus of the primer.

An alternative method, known as Genetic Bit Analysis or GBA TM is described by Goelet, P. et al. (PCT Appln. No. 92/15712). The method of Goelet, P. et al. uses mixtures of labeled terminators and a primer that is complementary to the sequence 3' to a polymorphic site. The labeled terminator that is incorporated is thus determined by, and complementary to, the nucleotide present in the polymorphic site of the target molecule being evaluated. In contrast to the method of Cohen et al. (French Patent 2,650,840; PCT Appln. No. WO91/02087) the method of Goelet, P. et al. is preferably a heterogeneous phase assay, in which the primer or the target molecule is immobilized to a solid phase.

Recently, several primer-guided nucleotide incorporation procedures for assaying polymorphic sites in DNA have been described (Komher, J. S. et al., Nucl. Acids. Res. 17:7779-7784 (1989); Sokolov, B. P., Nucl. Acids Res. 18:3671 (1990); Syvanen, A. -C., et al., Genomics 8:684-692 (1990), Kuppuswamy, M. N. et al., Proc. Natl. Acad. Sci. (U.S.A.) 88:1143-1147 (1991); Prezant, T. R. et al., Hum. Mutat. 1:159-164 (1992); Ugozzoli, L. et al., GATA 9:107-112 (1992); Nyren, P. et al., Anal. Biochem. 208:171-175 (1993)). These methods differ from GBA TM in that they all rely on the incorporation of labeled deoxynucleotides to discriminate between bases at a polymorphic site. In such a format, since the signal is proportional to the number of deoxynucleotides incorporated, polymorphisms that occur in runs of the same nucleotide can result in signals that are proportional to the length of the run (Syvanen, A.-C., et al., Amer. J. Hum. Genet. 52:46-59 (1993)).

For determining the identity of the allelic variant of a polymorphic region located in the coding region of a gene, yet other methods than those described above can be used. For example, identification of an allelic variant which encodes a mutated gene protein can be performed by using an antibody specifically recognizing the mutant protein in, e.g., immunohistochemistry or immunoprecipitation. Antibodies to wild-type gene protein are described, e.g., in Acton et al. (1999) Science 271:518 (antimouse gene antibody cross-reactive with human gene). Other antibodies to wild-type gene or mutated forms of gene proteins can be prepared according to methods known in the art. Alternatively, one can also measure an activity of an gene protein, such as binding to a lipid or lipoprotein. Binding assays are known in the art and involve, e.g., obtaining cells from a subject, and performing binding experiments with a labeled lipid, to determine whether binding to the mutated form of the receptor differs from binding to the wild-type of the receptor.

If a polymorphic region is located in an exon, either in a coding or non-coding region of the gene, the identity of the allelic variant can be determined by determining the molecular structure of the mRNA, pre-mRNA, or cDNA. The molecular structure can be determined using any of the above described methods for determining the molecular structure of the genomic DNA, e.g., sequencing and SSCP.

The methods described herein may be performed, for example, by utilizing prepackaged diagnostic kits, such as those described above, comprising at least one probe or primer nucleic acid described herein, which may be conveniently used, e.g., to determine whether a subject has or is at risk of developing a disease associated with a specific gene allelic variant.

Sample nucleic acid for using in the above-described diagnostic and prognostic methods can be obtained from any cell type or tissue of a subject. For example, a subject's bodily fluid (e.g. blood) can be obtained by known techniques (e.g. venipuncture) or from human tissues like heart (biopsies, transplanted organs). Alternatively, nucleic acid tests can be performed on dry samples (e.g. hair or skin). Fetal nucleic acid samples for prenatal diagnostics can be obtained from maternal blood as described in International Patent Application No.WO91/07660 to Bianchi. Alternatively, amniocytes or chorionic villi may be obtained for performing prenatal testing.

Diagnostic procedures may also be performed in situ directly upon tissue sections (fixed and/or frozen) of patient tissue obtained from biopsies or resections, such that no nucleic acid purification is necessary. Nucleic acid reagents may be used as probes and/or primers for such in situ procedures (see, for example, Nuovo, G. J., 1992, PCR in situ hybridization: protocols and applications, Raven Press, New York).

In addition to methods which focus primarily on the detection of one nucleic acid sequence, profiles may also be assessed in such detection schemes. Fingerprint profiles may be generated, for example, by utilizing a differential display procedure, Northern analysis and/or RT-PCR.

In practicing the present invention, the distribution of polymorphic patterns in a large number of individuals exhibiting particular markers of cardiovascular status or drug response is determined by any of the methods described above, and compared with the distribution of polymorphic patterns in patients that have been matched for age, ethnic origin, and/or any other statistically or medically relevant parameters, who exhibit quantitatively or qualitatively different status markers. Correlations are achieved using any method known in the art, including nominal logistic regression, chi square tests or standard least squares regression analysis. In this manner, it is possible to establish statistically significant correlations between particular polymorphic patterns and particular cardiovascular statuses (given in p values). It is further possible to establish statistically significant correlations between particular polymorphic patterns and changes in cardiovascular status or drug response such as, would result, e.g., from particular treatment regimens. In this manner, it is possible to correlate polymorphic patterns with responsivity to particular treatments.

In another embodiment of the present invention two or more polymorphic regions are combined to define so called 'haplotypes' . Haplotypes are groups of two or more SNPs that are functionally and/or spatially linked. It is possible to combine SNPs that are disclosed in the present invention either with each other or with additional polymorphic regions to form a haplotype. Haplotypes are expected to give better predictive/diagnostic information than a single SNP.

In a preferred embodiment of the present invention a panel of SNPs/haplotypes is defined that predicts the risk for CVD or drug response. This predictive panel is then used for genotyping of patients on a platform that can genotype multiple SNPs at the same time (Multiplexing). Preferred platforms are e.g. gene chips (Affymetrix) or the Luminex LabMAP reader. The subsequent identification and evaluation of a patient's haplotype can then help to guide specific and individualized therapy.

For example the present invention can identify patients exhibiting genetic polymorphisms or haplotypes which indicate an increased risk for adverse drug reactions. In that case the drug dose should be lowered in a way that the risk for ADR is diminished. Also if the patient's response to drug administration is particularly high (or the patient is badly metabolizing the drug), the drug dose should be lowered to avoid the risk of ADR.

In turn if the patient's response to drug administration is low (or the patient is a particularly high metabolizer of the drug), and there is no evident risk of ADR, the drug dose should be raised to an efficacious level.

It is self evident that the ability to predict a patient's individual drug response should affect the formulation of a drug, i.e. drug formulations should be tailored in a way that they suit the different patient classes (low/high responder, poor/good metabolizer, ADR prone patients). Those different drug formulations may encompass different doses of the drug, i.e. the medicinal products contains low or high amounts of the active substance. In another embodiement of the invention the drug formulation may contain additional substances that facilitate the beneficial effects and/or diminish the risk for ADR (Folkers et al. 1991, US Pat. 5,316,765).

### Isolated Polymorphic Nucleic Acids, Probes, and Vectors

The present invention provides isolated nucleic acids comprising the polymorphic positions described herein for human genes; vectors comprising the nucleic acids; and transformed host cells comprising the vectors. The invention also provides probes which are useful for detecting these polymorphisms.

In practicing the present invention, many conventional techniques in molecular biology, microbiology, and recombinant DNA, are used. Such techniques are well known and are explained fully in, for example, Sambrook et al., 1989, Molecular Cloning: A Laboratory Manual, Second Edition, Cold Spring Harbor Laboratory Press, Cold Spring Harbor, New York; DNA Cloning: A Practical Approach, Volumes I and II, 1985 (D. N. Glover ed.); Oligonucleotide Synthesis, 1984, (M. L.Gait ed.); Nucleic Acid Hybridization, 1985, (Hames and Higgins); Ausubel et al., Current Protocols in Molecular Biology, 1997, (John Wiley and Sons); and Methods in Enzymology Vol. 154 and Vol. 155 (Wu and Grossman, and Wu, eds., respectively).

Insertion of nucleic acids (typically DNAs) comprising the sequences in a functional surrounding like full length cDNA of the present invention into a vector is easily accomplished when the termini of both the DNAs and the vector comprise compatible restriction sites. If this cannot be done, it may be necessary to modify the termini of the DNAs and/or vector by digesting back single-stranded DNA overhangs generated by restriction endonuclease cleavage to produce blunt ends, or to achieve the same result by filling in the single-stranded termini with an appropriate DNA polymerase.

Alternatively, any site desired may be produced, e.g., by ligating nucleotide sequences (linkers) onto the termini. Such linkers may comprise specific oligonucleotide sequences that define desired restriction sites. Restriction sites can also be generated by the use of the polymerase chain reaction (PCR). See, e.g., Saiki et al., 1988, Science 239:48. The cleaved vector and the DNA fragments may also be modified if required by homopolymeric tailing.

The nucleic acids may be isolated directly from cells or may be chemically synthesized using known methods. Alternatively, the polymerase chain reaction (PCR) method can be used to produce the nucleic acids of the invention, using either chemically synthesized strands or genomic material as templates. Primers used for PCR can be synthesized using the sequence information provided herein and can further be designed to introduce appropriate new restriction sites, if desirable, to facilitate incorporation into a given vector for recombinant expression.

The nucleic acids of the present invention may be flanked by native gene sequences, or may be associated with heterologous sequences, including promoters, enhancers, response elements, signal sequences, polyadenylation sequences, introns, 5'- and 3'-noncoding regions, and the like. The nucleic acids may also be modified by many means known in the art. Non-limiting examples of such modifications include methylation, "caps", substitution of one or more of the naturally occurring nucleotides with an analog, internucleotide modifications such as, for example, those with uncharged linkages (e.g., methyl phosphonates, phosphotriesters, phosphoroamidates, carbamates, morpholines etc.) and with charged linkages (e.g., phosphorothioates, phosphorodithioates, etc.). Nucleic acids may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. PNAs are also included. The nucleic acid may be derivatized by formation of a methyl or ethyl phosphotriester or an alkyl phosphoramidate linkage. Furthermore, the nucleic acid sequences of the present invention may also be modified with a label capable of providing a detectable signal, either directly or indirectly. Exemplary labels include radioisotopes, fluorescent molecules, biotin, and the like.

The invention also provides nucleic acid vectors comprising the gene sequences or derivatives or fragments thereof of genes described in the Examles. A large number of vectors, including plasmid and fungal vectors, have been described for replication and/or expression in a variety of eukaryotic and prokaryotic hosts, and may be used for gene therapy as well as for simple cloning or protein expression. Non-limiting examples of suitable vectors include without limitation pUC plasmids, pET plasmids (Novagen, Inc., Madison, Wis.), or pRSET or pREP (Invitrogen, San Diego, Calif.), and many appropriate host cells, using methods disclosed or cited herein or otherwise known to those skilled in the relevant art. The particular choice of vector/host is not critical to the practice of the invention.

Suitable host cells may be transformed/transfected/infected as appropriate by any suitable method including electroporation, CaCl₂ mediated DNA uptake, fungal or viral infection, microinjection, microprojectile, or other established methods. Appropriate host cells included bacteria, archebacteria, fungi, especially yeast, and plant and animal cells, especially mammalian cells. A large number of transcription initiation and termination regulatory regions have been isolated and shown to be effective in the transcription and translation of heterologous proteins in the various hosts. Examples of these regions, methods of isolation, manner of manipulation, etc. are known in the art. Under appropriate expression conditions, host cells can be used as a source of recombinantly produced peptides and polypeptides encoded by genes of the Examples. Nucleic acids encoding peptides or polypeptides from gene sequences of the Examples may also be introduced into cells by recombination events. For example, such a sequence can be introduced into a cell and thereby effect homologous recombination at the site of an endogenous gene or a sequence with substantial identity to the gene. Other recombination-based methods such as non-homologous recombinations or deletion of endogenous genes by homologous recombination may also be used.

In case of proteins that form heterodimers or other multimers, both or all subunits have to be expressed in one system or cell.

The nucleic acids of the present invention find use as probes for the detection of genetic polymorphisms and as templates for the recombinant production of normal or variant peptides or polypeptides encoded by genes listed in the Examples.

Probes in accordance with the present invention comprise without limitation isolated nucleic acids of about 10-100 bp, preferably 15-75 bp and most preferably 17-25 bp in length, which hybridize at high stringency to one or more of the polymorphic sequences disclosed herein or to a sequence immediately adjacent to a polymorphic position. Furthermore, in some embodiments a full-length gene sequence may be used as a probe. In one series of embodiments, the probes span the polymorphic positions in genes disclosed herein. In another series of embodiments, the probes correspond to sequences immediately adjacent to the polymorphic positions.

### Polymorphic Polypeptides and Polymorphism-Specific Antibodies

The present invention encompasses isolated peptides and polypeptides encoded by genes listed in the Examples comprising polymorphic positions disclosed herein. In one preferred embodiment, the peptides and polypeptides are useful screening targets to identify cardiovascular drugs. In another preferred embodiments, the peptides and polypeptides are capable of eliciting antibodies in a suitable host animal that react specifically with a polypeptide comprising the polymorphic position and distinguish it from other polypeptides having a different sequence at that position.

Polypeptides according to the invention are preferably at least five or more residues in length, preferably at least fifteen residues. Methods for obtaining these polypeptides are described below. Many conventional techniques in protein biochemistry and immunology are used. Such techniques are well known and are explained in Immunochemical Methods in Cell and Molecular Biology, 1987 (Mayer and Waler, eds; Academic Press, London); Scopes, 1987, Protein Purification: Principles and Practice, Second Edition (Springer-Verlag, N.Y.) and Handbook of Experimental Immunology, 1986, Volumes I-IV (Weir and Blackwell eds.).

Nucleic acids comprising protein-coding sequences can be used to direct the ITT recombinant expression of polypeptides encoded by genes disclosed herein in intact cells or in cell-free translation systems. The known genetic code, tailored if desired for more efficient expression in a given host organism, can be used to synthesize oligonucleotides encoding the desired amino acid sequences. The polypeptides may be isolated from human cells, or from heterologous organisms or cells (including, but not limited to, bacteria, fungi, insect, plant, and mammalian cells) into which an appropriate protein-coding sequence has been introduced and expressed. Furthermore, the polypeptides may be part of recombinant fusion proteins.

Peptides and polypeptides may be chemically synthesized by commercially available automated procedures, including, without limitation, exclusive solid phase synthesis, partial solid phase methods, fragment condensation or classical solution synthesis. The polypeptides are preferably prepared by solid phase peptide synthesis as described by Merrifield, 1963, J. Am. Chem. Soc. 85:2149.

Methods for polypeptide purification are well-known in the art, including, without limitation, preparative disc-gel electrophoresis, isoelectric focusing, HPLC, reversed-phase HPLC, gel filtration, ion exchange and partition chromatography, and countercurrent distribution. For some purposes, it is preferable to produce the polypeptide in a recombinant system in which the protein contains an additional sequence tag that facilitates purification, such as, but not limited to, a polyhistidine sequence. The polypeptide can then be purified from a crude lysate of the host cell by chromatography on an appropriate solid-phase matrix. Alternatively, antibodies produced against peptides encoded by genes disclosed herein, can be used as purification reagents. Other purification methods are possible.

The present invention also encompasses derivatives and homologues of the polypeptides. For some purposes, nucleic acid sequences encoding the peptides may be altered by substitutions, additions, or deletions that provide for functionally equivalent molecules, i.e., function-conservative variants. For example, one or more amino acid residues within the sequence can be substituted by another amino acid of similar properties, such as, for example, positively charged amino acids (arginine, lysine, and histidine); negatively charged amino acids (aspartate and glutamate); polar neutral amino acids; and non-polar amino acids.

The isolated polypeptides may be modified by, for example, phosphorylation, sulfation, acylation, or other protein modifications. They may also be modified with a label capable of providing a detectable signal, either directly or indirectly, including, but not limited to, radioisotopes and fluorescent compounds.

The present invention also encompasses antibodies that specifically recognize the polymorphic positions of the invention and distinguish a peptide or polypeptide containing a particular polymorphism from one that contains a different sequence at that position. Such polymorphic position-specific antibodies according to the present invention include polyclonal and monoclonal antibodies. The antibodies may be elicited in an animal host by immunization with peptides encoded by genes disclosed herein or may be formed by in vitro immunization of immune cells. The immunogenic components used to elicit the antibodies may be isolated from human cells or produced in recombinant systems. The antibodies may also be produced in recombinant systems programmed with appropriate antibody-encoding DNA. Alternatively, the antibodies may be constructed by biochemical reconstitution of purified heavy and light chains. The antibodies include hybrid antibodies (i.e., containing two sets of heavy chain/light chain combinations, each of which recognizes a different antigen), chimeric antibodies (i.e., in which either the heavy chains, light chains, or both, are fusion proteins), and univalent antibodies (i.e., comprised of a heavy chain/light chain complex bound to the constant region of a second heavy chain). Also included are Fab fragments, including Fab' and F(ab).sub.2 fragments of antibodies. Methods for the production of all of the above types of antibodies and derivatives are well-known in the art and are discussed in more detail below. For example, techniques for producing and processing polyclonal antisera are disclosed in Mayer and Walker, 1987, Immunochemical Methods in Cell and Molecular Biology, (Academic Press, London). The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. See, e.g., Schreier et al., 1980, Hybridoma Techniques; U.S. Pat. Nos. 4,341,761; 4,399,121; 4,427,783; 4,444,887; 4,466,917; 4,472,500; 4,491,632; and 4,493,890. Panels of monoclonal antibodies produced against peptides encoded by genes disclosed herein can be screened for various properties; i.e. for isotype, epitope affinity, etc.

The antibodies of this invention can be purified by standard methods, including but not limited to preparative disc-gel electrophoresis, isoelectric focusing, HPLC, reversed-phase HPLC, gel filtration, ion exchange and partition chromatography, and countercurrent distribution. Purification methods for antibodies are disclosed, e.g., in The Art of Antibody Purification, 1989, Amicon Division, W. R. Grace & Co. General protein purification methods are described in Protein Purification: Principles and Practice, R. K. Scopes, Ed., 1987, Springer-Verlag, New York, N.Y.

Methods for determining the immunogenic capability of the disclosed sequences and the characteristics of the resulting sequence-specific antibodies and immune cells are well-known in the art. For example, antibodies elicited in response to a peptide comprising a particular polymorphic sequence can be tested for their ability to specifically recognize that polymorphic sequence, i.e., to bind differentially to a peptide or polypeptide comprising the polymorphic sequence and thus distinguish it from a similar peptide or polypeptide containing a different sequence at the same position.

### Kits

As set forth herein, the invention provides diagnostic methods, e.g., for determining the identity of the allelic variants of polymorphic regions present in the gene loci of genes disclosed herein, wherein specific allelic variants of the polymorphic region are associated with cardiovascular diseases. In a preferred embodiment, the diagnostic kit can be used to determine whether a subject is at risk of developing a cardiovascular disease. This information could then be used, e.g., to optimize treatment of such individuals.

In preferred embodiments, the kit comprises a probe or primer which is capable of hybridizing to a gene and thereby identifying whether the gene contains an allelic variant of a polymorphic region which is associated with a risk for cardiovascular disease. The kit preferably further comprises instructions for use in diagnosing a subject as having, or having a predisposition, towards developing a cardiovascular disease. The probe or primers of the kit can be any of the probes or primers described in this file.

Preferred kits for amplifying a region of a gene comprising a polymorphic region of interest comprise one, two or more primers.

### Antibody-based diagnostic methods and kits:

The invention also provides antibody-based methods for detecting polymorphic patterns in a biological sample. The methods comprise the steps of: (i) contacting a sample with one or more antibody preparations, wherein each of the antibody preparations is specific for a particular polymorphic form of the proteins encoded by genes disclosed herein, under conditions in which a stable antigen-antibody complex can form between the antibody and antigenic components in the sample; and (ii) detecting any antigen-antibody complex formed in step (i) using any suitable means known in the art, wherein the detection of a complex indicates the presence of the particular polymorphic form in the sample.

Typically, immunoassays use either a labelled antibody or a labelled antigenic component (e.g., that competes with the antigen in the sample for binding to the antibody). Suitable labels include without limitation enzyme-based, fluorescent, chemiluminescent, radioactive, or dye molecules. Assays that amplify the signals from the probe are also known, such as, for example, those that utilize biotin and avidin, and enzyme-labelled immunoassays, such as ELISA assays.

The present invention also provides kits suitable for antibody-based diagnostic applications. Diagnostic kits typically include one or more of the following components:
(i) Polymorphism-specific antibodies. The antibodies may be pre-labelled; alternatively, the antibody may be unlabelled and the ingredients for labelling may be included in the kit in separate containers, or a secondary, labelled antibody is provided; and
(ii) Reaction components: The kit may also contain other suitably packaged reagents and materials needed for the particular immunoassay protocol, including solid-phase matrices, if applicable, and standards.

The kits referred to above may include instructions for conducting the test. Furthermore, in preferred embodiments, the diagnostic kits are adaptable to high-throughput and/or automated operation.

### Drug Targets and Screening Methods

According to the present invention, nucleotide sequences derived from genes disclosed herein and peptide sequences encoded by genes disclosed herein, particularly those that contain one or more polymorphic sequences, comprise useful targets to identify cardiovascular drugs, i.e., compounds that are effective in treating one or more clinical symptoms of cardiovascular disease. Furthermore, especially when a protein is a multimeric protein that are build of two or more subunits, is a combination of different polymorphic subunits very useful.

Drug targets include without limitation (i) isolated nucleic acids derived from the genes disclosed herein, and (ii) isolated peptides and polypeptides encoded by genes disclosed herein, each of which comprises one or more polymorphic positions.

### In vitro screening methods:

In one series of embodiments, an isolated nucleic acid comprising one or more polymorphic positions is tested in vitro for its ability to bind test compounds in a sequence-specific manner. The methods comprise:
(i) providing a first nucleic acid containing a particular sequence at a polymorphic position and a second nucleic acid whose sequence is identical to that of the first nucleic acid except for a different sequence at the same polymorphic position;
(ii) contacting the nucleic acids with a multiplicity of test compounds under conditions appropriate for binding; and
(iii) identifying those compounds that bind selectively to either the first or second nucleic acid sequence.

Selective binding as used herein refers to any measurable difference in any parameter of binding, such as, e.g., binding affinity, binding capacity, etc.

In another series of embodiments, an isolated peptide or polypeptide comprising one or more polymorphic positions is tested in vitro for its ability to bind test compounds in a sequence-specific manner. The screening methods involve:
(i) providing a first peptide or polypeptide containing a particular sequence at a polymorphic position and a second peptide or polypeptide whose sequence is identical to the first peptide or polypeptide except for a different sequence at the same polymorphic position;
(ii) contacting the polypeptides with a multiplicity of test compounds under conditions appropriate for binding; and
(iii) identifying those compounds that bind selectively to one of the nucleic acid sequences.

In preferred embodiments, high-throughput screening protocols are used to survey a large number of test compounds for their ability to bind the genes or peptides disclosed above in a sequence-specific manner.

Test compounds are screened from large libraries of synthetic or natural compounds. Numerous means are currently used for random and directed synthesis of saccharide, peptide, and nucleic acid based compounds. Synthetic compound libraries are commercially available from Maybridge Chemical Co. (Trevillet, Cornwall, UK), Comgenex (Princeton, N.J.), Brandon Associates (Merrimack, N.H.), and Microsource (New Milford, Conn.). A rare chemical library is available from Aldrich (Milwaukee, Wis.). Alternatively, libraries of natural compounds in the form of bacterial, fungal, plant and animal extracts are available from e.g. Pan Laboratories (Bothell, Wash.) or MycoSearch (N.C.), or are readily producible. Additionally, natural and synthetically produced libraries and compounds are readily modified through conventional chemical, physical, and biochemical means.

### In vivo screening methods:

Intact cells or whole animals expressing polymorphic variants of genes disclosed herein can be used in screening methods to identify candidate cardiovascular drugs.

In one series of embodiments, a permanent cell line is established from an individual exhibiting a particular polymorphic pattern. Alternatively, cells (including without limitation mammalian, insect, yeast, or bacterial cells) are programmed to express a gene comprising one or more polymorphic sequences by introduction of appropriate DNA. Identification of candidate compounds can be achieved using any suitable assay, including without limitation (i) assays that measure selective binding of test compounds to particular polymorphic variants of proteins encoded by genes disclosed herein; (ii) assays that measure the ability of a test compound to modify (i.e., inhibit or enhance) a measurable activity or function of proteins encoded by genes disclosed herein; and (iii) assays that measure the ability of a compound to modify (i.e., inhibit or enhance) the transcriptional activity of sequences derived from the promoter (i.e., regulatory) regions of genes disclosed herein.

In another series of embodiments, transgenic animals are created in which (i) one or more human genes disclosed herein, having different sequences at particular polymorphic positions are stably inserted into the genome of the transgenic animal; and/or (ii) the endogenous genes disclosed herein are inactivated and replaced with human genes disclosed herein, having different sequences at particular polymorphic positions. See, e.g., Coffman, Semin. Nephrol. 17:404, 1997; Esther et al., Lab. Invest. 74:953, 1996; Murakami et al., Blood Press. Suppl. 2:36, 1996. Such animals can be treated with candidate compounds and monitored for one or more clinical markers of cardiovascular status.

The following are intended as non-limiting examples of the invention.

### Material and Methods

Genotyping of patient DNA with the Pyrosequencing^{TM} Method as described in the patent application WO 9813523:

First a PCR is set up to amplify the flanking regions around a SNP. Therefor 2 ng of genomic DNA (patient sample) are mixed with a primerset (20 - 40 pmol) producing a 75 to 320 bp PCR fragment with 0,3 to 1 U Qiagens Hot Star Taq Polymerase^{TM} in a total volume of 20 µL. One primer is biotinylated depending on the direction of the sequencing primer. To force the biotinylated primer to be incorporated it is used 0,8 fold.

For primer design, programms like Oligo 6^{TM} (Molecular Biology Insights) or Primer Select^{TM} (DNAStar) are used. PCR setup is performed by a BioRobot 3000 ^{TM} from Qiagen. PCR takes place in T1 or Tgradient Thermocyclers ^{TM} from Biometra.

The whole PCR reaction is transferred into a PSQ plate ^{TM} (Pyrosequencing) and prepared using the Sample Prep Tool ^{TM} and SNP Reagent Kit ^{TM} from Pyrosequencing according to their instructions.

### Preparation of template for Pyrosequencing^{TM}:

### Sample preparation using PSQ 96 Sample Prep Tool:

1. Mount the PSQ 96 Sample Prep Tool Cover onto the PSQ 96 Sample Prep Tool as follows: Place the cover on the desk, retract the 4 attachment rods by separating the handle from the magnetic rod holder, fit the magnetic rods into the holes of the cover plate, push the handle downward until a click is heard. The PSQ 96 Sample Prep Tool is now ready for use.
2. To transfer beads from one plate to another, place the covered tool into the PSQ 96 Plate containing the samples and lower the magnetic rods by separating the handle from the magnetic rod holder. Move the tool up and down a few times then wait for 30-60 seconds. Transfer the beads into a new PSQ 96 plate containing the solution of choice.
3. Release the beads by lifting the magnetic rod holder, bringing it together with the handle. Move the tool up and down a few times to make sure that the beads are released.

All steps are performed at room temperature unless otherwise stated.

### Immobilization of PCR product:

Biotinylated PCR products are immobilized on streptavidin-coated Dynabeads ^{TM} M-280 Streptavidin. Parallel immobilization of several samples are performed in the PSQ 96 Plate.
1. Mix PCR product, 20 µl of a well optimized PCR, with 25 µl 2X BW-buffer II. Add 60-150 µg Dynabeads. It is also possible to add a mix of Dynabeads and 2X BW-buffer II to the PCR product yielding a final BW-buffer II concentration of approximately 1x.
2. Incubate at 65°C for 15 min agitation constantly to keep the beads dispersed. For optimal immobilization of fragments longer than 300 bp use 30 min incubation time.

### Strand separation:

3. For strand separation, use the PSQ 96 Sample Prep Tool to transfer the beads with the immobilized sample to a PSQ 96 Plate containing 50 µl 0.50 M NaOH per well. Release the beads.
4. After approximately 1 min, transfer the beads with the immobilized strand to a PSQ 96 Plate containing 99 µl 1x Annealing buffer per well and mix thoroughly.
5. Transfer the beads to a PSQ 96 Plate containing 45 µl of a mix of 1x Annealing buffer and 3-15 pmoles sequencing primer per well.
6. Heat at 80°C for 2 minutes in the PSQ 96 Sample Prep Thermoplate and move to room temperature.
7. After reaching room temperature, continue with the sequencing reaction.

### Sequencing reaction:

1. Choose the method to be used ("SNP Method") and enter relevant information in the PSQ 96 Instrument Control software.
2. Place the cartridge and PSQ 96 Plate in the PSQ 96 Instrument.
3. Start the run.

### Genotyping using the ABI 7700/7900 instrument (TaqMan)

SNP genotypisation using the TaqMan (Applied Biosystems/Perkin Elmer) was performed according to the manufacturer's instructions. The TaqMan assay is discussed by Lee et al., Nucleic Acids Research 1993, 21: 3761-3766.

### Genotyping with a service contractor:

Qiagen Genomics, formerly Rapigene, is a service contractor for genotyping SNPs in patient samples. Their method is based on a primer extension method where two complementary primers are designed for each genotype that are labeled with different tags. Depending on the genotype only one primer will be elongated together with a certain tag. This tag can be detected with mass spectrometry and is a measure for the respective genotype. The method is described in the following patent: "Detection and identification of nucleic acid molecules - using tags which may be detected by non-fluorescent spectrometry or potentiometry" (WO 9727325).

### Examples

To exemplify the present invention and it's utility baySNP 2214 will be used in the following:
baySNP 2214 is an A to T polymorphism and presumably resides in the gene of the human ISG-54K gene (interferon stimulated gene) encoding a 54 kDA protein (information taken from table 3). baySNP 2214 was genotyped in various patient cohorts using the primers from table 2. As a result the following number of patients carrying different genotypes were found (information combined from tables 3 and 5a):

| **baySNP** | **Cohort** | **Total** | **Geno type 11 "AA"** | **Geno-type 12 "AT"** | **Geno-type 22 "TT"** |
|---|---|---|---|---|---|
| 2214 | HELD_MAL_CASE | 14 | 3 | 9 | 2 |
| 2214 | HELD_MAL_CTRL | 18 | 0 | 11 | 7 |

When comparing the number of male patients exhibiting CVD (HELD_MAL_CASE) with the control cohort (HELD_MAL_CTRL) it appears that the number of CVD patients carrying the AA genotype is increased, whereas the number of CVD patients carrying the TT genotype is diminished. This points to higher risk of CVD among male individuals with the CC genotype. Applying statistical tests on those findings the following p-values were obtained (data taken from table 5b):

| **BAYSNP** | **COMPARISON** | **GTYPE CPVAL** | **GTYPE XPVAL** | **GTYPE LRPVAL** |
|---|---|---|---|---|
| 2214 | HELD_MAL_CC | 0,0619 | 0,0799 | 0,0334 |

As at least one of the GTYPE p values is below 0,05 the association of genotype and CVD is regarded as statistically significant. I.e. the analysis of a patient's genotype can predict the risk to suffer from CVD. In more detail one can calculate the relative risk to suffer from CVD when carrying a certain genotype (data taken from table 6a):

| **BAYSNP** | **COMPARISON** | **GTYPE1** | **GTYPE2** | **GTYPE3** | **RR1** | **RR2** | **RR3** |
|---|---|---|---|---|---|---|---|
| 2214 | HELD_MAL_CC | AA | AT | TT | 2,64 | 1,08 | 0,43 |

In case of baySNP 2214 the risk to suffer from CVD is 2,64 times higher when carrying the AA genotype. This indicates that a AA polymorphism in baySNP 2214 is an independent risk factor for CVD. On the other hand carriers of a TT genotype have a reduced risk to suffer from CVD.

In addition statistical associations can be calculated on the basis on alleles. This calculation would identify risk alleles instead of risk genotypes.

In case of baySNP 900073 the following allele counts were obtained (data combined from tables 3 and 5a):

| **baySNP** | **Cohort** | **Total** | **Allele 1 "C"** | **Allele 2 "G"** |
|---|---|---|---|---|
| 900073 | CVD_MAL_CASE | 69 | 47 | 91 |
| 900073 | CVD_MAL_CTRL | 32 | 12 | 52 |

When comparing the number of male patients with CVD (CVD_MAL_CASE) with the control cohort (CVD_MAL_CTRL) it appears that the number of CVD patients carrying the C allele is increased, whereas the number of CVD patients carrying the G allele is diminished. This points to a higher risk for CVD among male individuals with the C allele. When applying statistical tests on those findings the following p-values were obtained (data taken from table 5b):

| **BAYSNP** | **COMPARISON** | **ALLELE CPVAL** | **ALLELE XPVAL** | **ALLELE LRPVAL** |
|---|---|---|---|---|
| 900073 | CVD_MAL | 0,026 | 0,0306 | 0,0224 |

As at least one of the ALLELE p values is below 0,05 the association of allele and CVD risk is regarded as statistically significant (in this example significant p values were obtained from all three statistical tests). I.e. also the analysis of a patient's alleles from baySNP 900073 can predict the risk to suffer from CVD. In more detail one can calculate the relative risk to suffer from CVD when carrying a certain allele (data taken from table 6b):

| **baySNP** | **Allele 1** | **Allele 2** | **COMPARISON** | **RR1** | **RR2** |
|---|---|---|---|---|---|
| 900073 | C | G | CVD_MAL | 1,25 | 0,8 |

In case of baySNP 900073 the risk to suffer from CVD for males is 1,25 times higher when carrying the C allele. This indicates that the C allele of baySNP900073 is an independent risk factor for CVD in males.

Another example is baySNP 4564, which is taken to exemplify polymorphisms relevant for adverse drug reactions. baySNP 4564 was found significant when comparing male patients with advanced ADR to the respective controls (as defined in table 1b).

The relative risk ratios for the genotypes AA, AG and GG were as follows (data taken from table 6a):

| **BAYSNP** | **COMPARISON** | **GTYPE1** | **GTYPE2** | **GTYPE3** | **RR1** | **RR2** | **RR3** |
|---|---|---|---|---|---|---|---|
| 4564 | HELD_MAL_ADR3ULN | AA | AG | GG | 0,49 | 2,05 | null |

In this case male patients carrying the AG genotype have a 2,05 times higher risk to suffer from ADR. In other words those patients should either receive lower doses of statins or switch to an alternative therapy in order to avoid ADR. On the other hand male patients with the AA genotype appear to be more resistant to ADR and hence better tolerate statin therapy.

As a last example baySNP 8589 is taken: In that case the risk to exhibit a high responder phenotype is 1,31 times higher when carrying the C allele. This indicates that the C allele of baySNP 8589 is an independent risk factor for a high statin response in females. In other words those patients should receive lower doses of statins in order to avoid ADR. However due to their 'high responder' phenotype they will still benefit from the drug. In turn carriers of the T allele should receive higher drug doses in order to experience a benefical therapeutic effect

**Table 1a**

| Definition of "good" and "bad" serum lipid levels | | |
|---|---|---|
| | "Good" | "Bad" |
| LDL-Cholesterol [mg/dL] | 125 -150 | 170 - 200 |
| Cholesterol [mg/dL] | 190 - 240 | 265 - 315 |
| HDL-Cholesterol [mg/dL] | 60 -105 | 30 - 55 |
| Triglycerides [mg/dL] | 45 - 115 | 170 - 450 |

**Table 1b**

| Definition of drug response phenotypes | |
|---|---|
| Low responder | Decrease of serum LDL of at least 10% and at most 50% upon administration of 0.8 mg Cerivastatin (female patients) |
| High responder | Decrease of serum LDL of at least 50% upon administration of 0.4 mg Cerivastatin (female patients) |
| Very low responder | Decrease of serum LDL of at least 10% and at most 35% upon administration of 0.8 mg Cerivastatin (female patients) |
| Very high responder | Decrease of serum LDL of at least 55% upon administration of 0.4 mg Cerivastatin (female patients) |
| Ultra low responder | Decrease of serum LDL of at least 10% and at most 25% upon administration of 0.8 mg Cerivastatin (female patients) |
| Ultra high responder | Decrease of serum LDL of at least 60% upon administration of 0.4 mg Cerivastatin (female patients) |
| Tolerant patient | No diagnosis of muscle cramps, muscle pain, muscle weakness, myalgia or myopathy AND serum CK levels below 70 mg/dl in women and below 80 mg/dl in men. |
| ADR patient (CK increase at least 2xULN) | Diagnosis of muscle cramps, muscle pain, muscle weakness, myalgia or myopathy OR serum CK levels higher than 140 mg/dl in women and 160 mg/dl in men. |
| Advanced ADR patient [ADR3] (advanced CK increase, at least 3×ULN)* | Serum CK levels higher than 210 mg/dl in women and 240 mg/dl in men |
| Severe ADR patient [ADR5] (severe CK increase, at least 5×ULN)* | Serum CK levels higher than 350 mg/dl in women and 400 mg/dl in men |

| | |
|---|---|
| * : When assembling the cohorts for advanced and severe ADR we focused on the CK serum levels as those provide a more independent measure of statin related ADR. | |

**Table 1c**

| Definition of "high" and "low" serum HDL cholesterol levels | | |
|---|---|---|
| | Male individuals | Female individuals |
| ,High' HDL-Cholesterol [mg/dL] | >=80 | >=104 |
| ,Low' HDL-Cholesterol [mg/dL] | <=35 | <=37 |

An informed consent was signed by the patients and control people. Blood was taken by a physician according to medical standard procedures.

Samples were collected anonymous and labeled with a patient number.
DNA was extracted using kits from Qiagen.

### Table 2a Oligonucleotide primers used for genotyping using mass spectrometry

The baySNP number refers to an internal numbering of the PA SNPs. Primer sequences are listed for preamplification of the genomic fragments (primers EF and ER) and for subsequent allele specific PCR of the SNP.

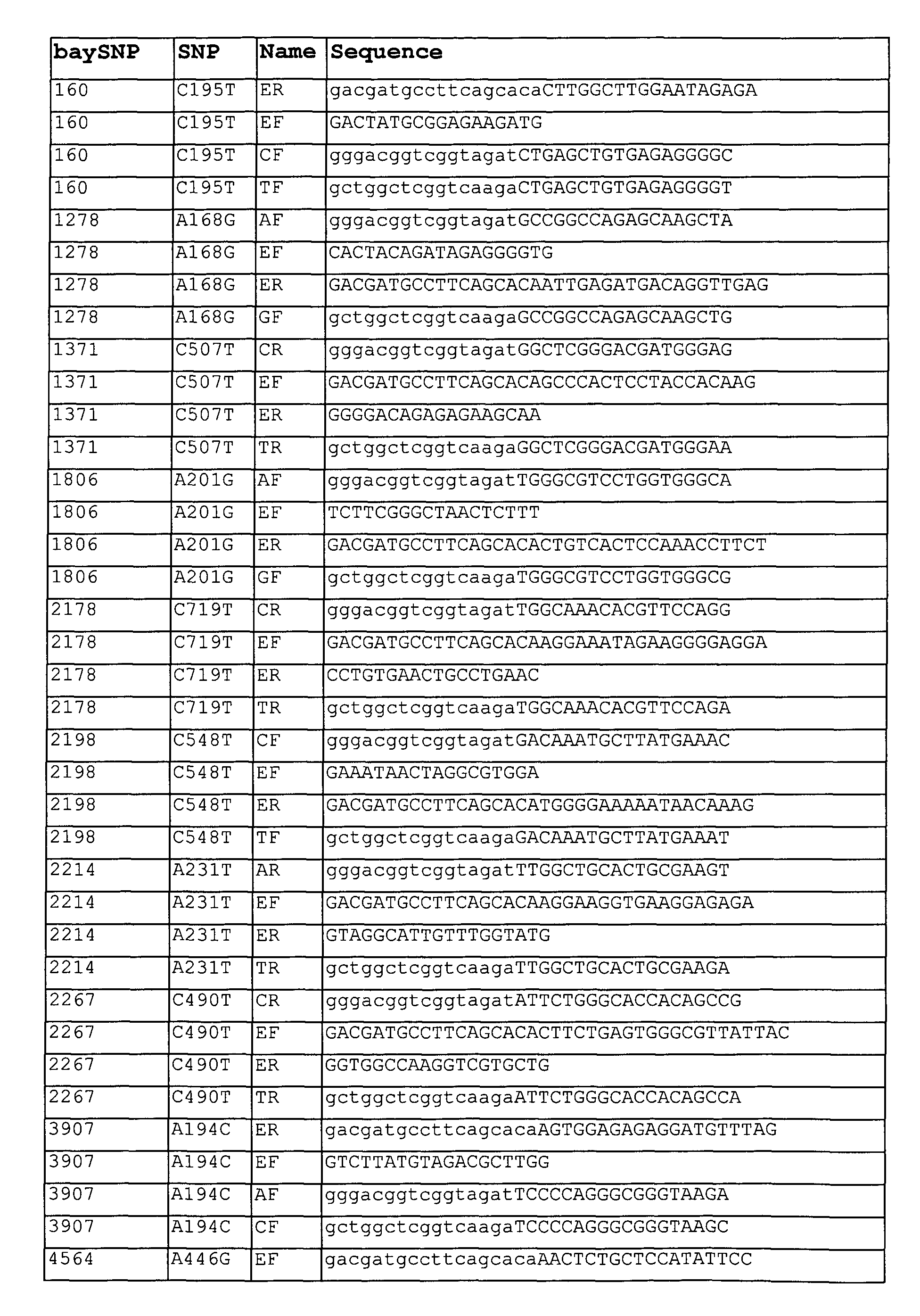

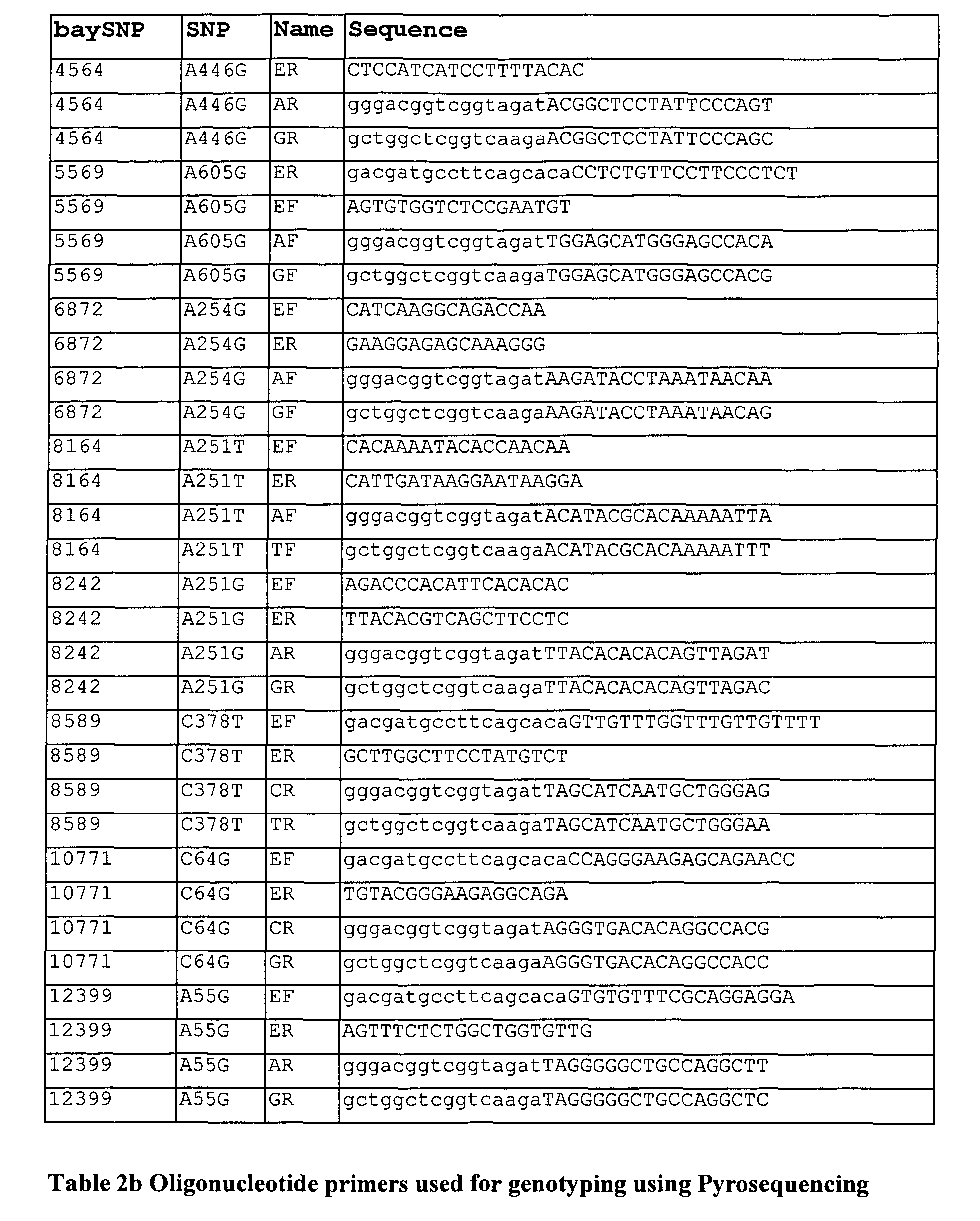

The baySNP number refers to an internal numbering of the PA SNPs. Primer sequences are listed for preamplification of the genomic fragments and for sequencing of the SNP using the pyrosequencing method. Bio: Biotinylated Oligonucleotide.

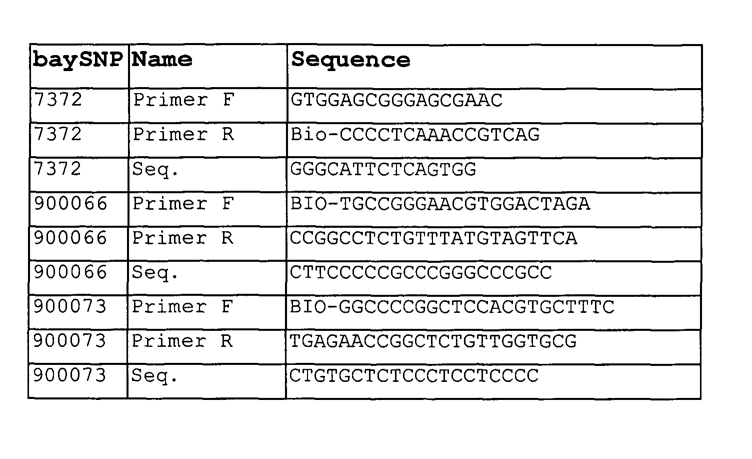

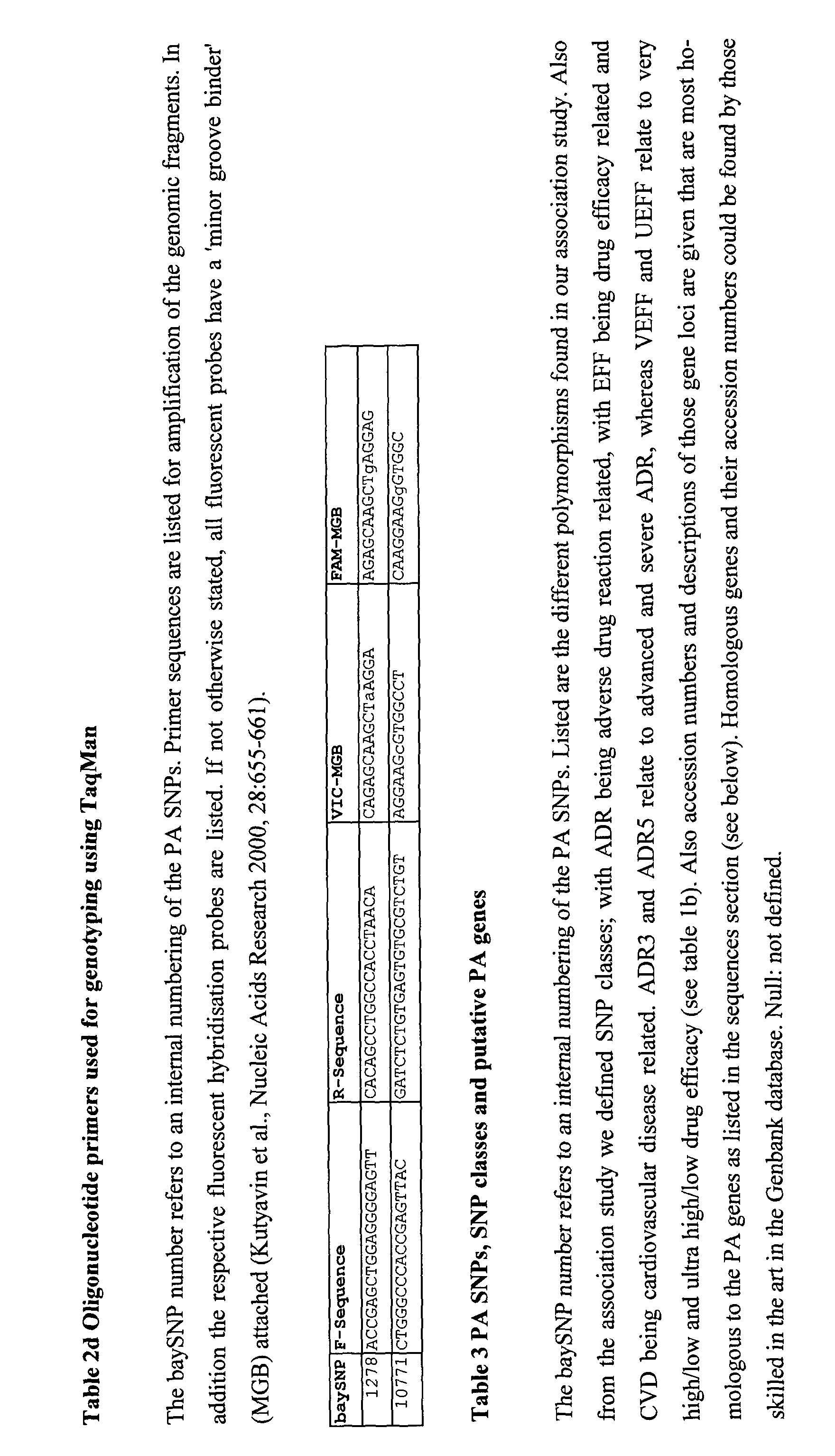

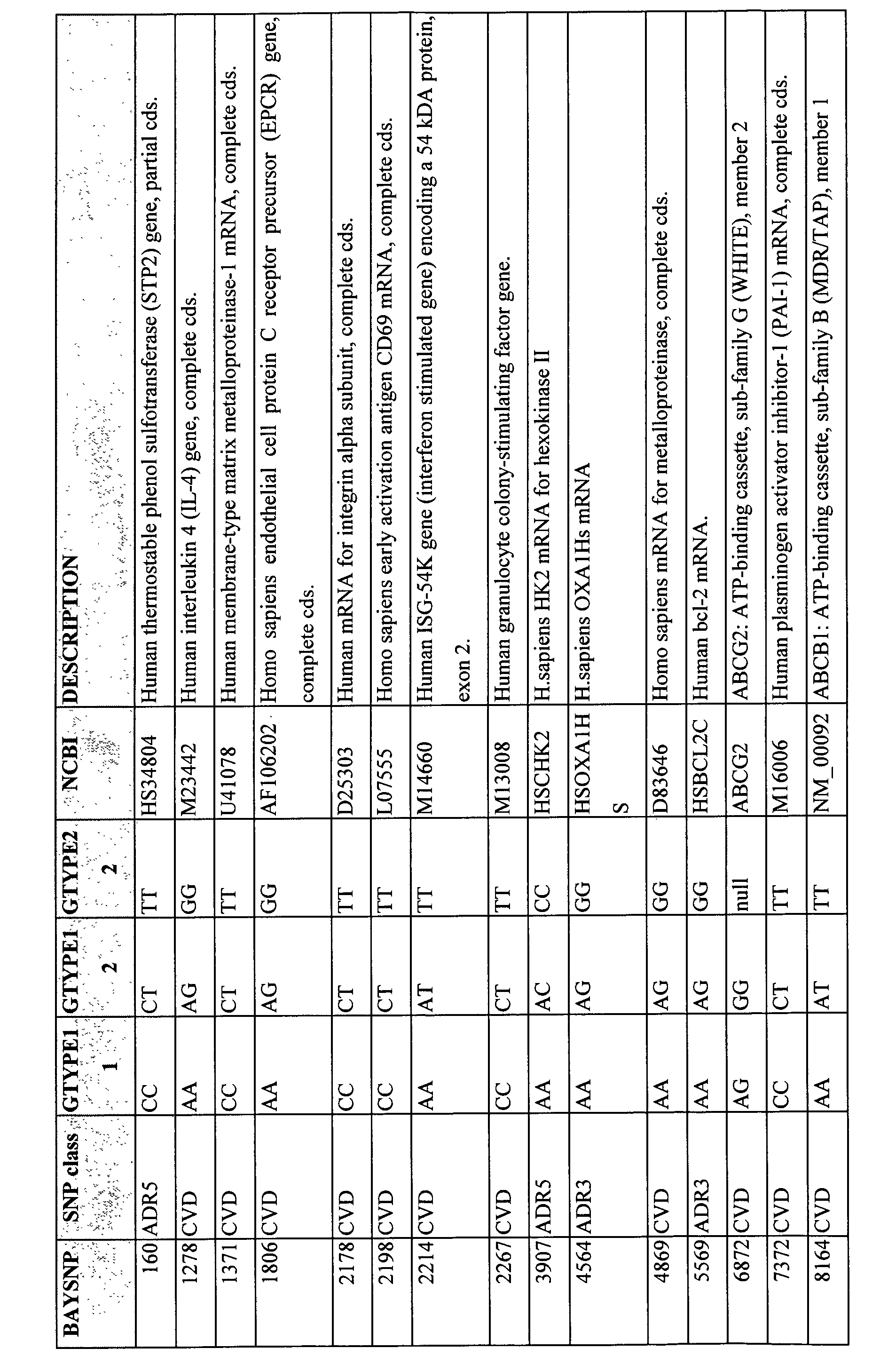

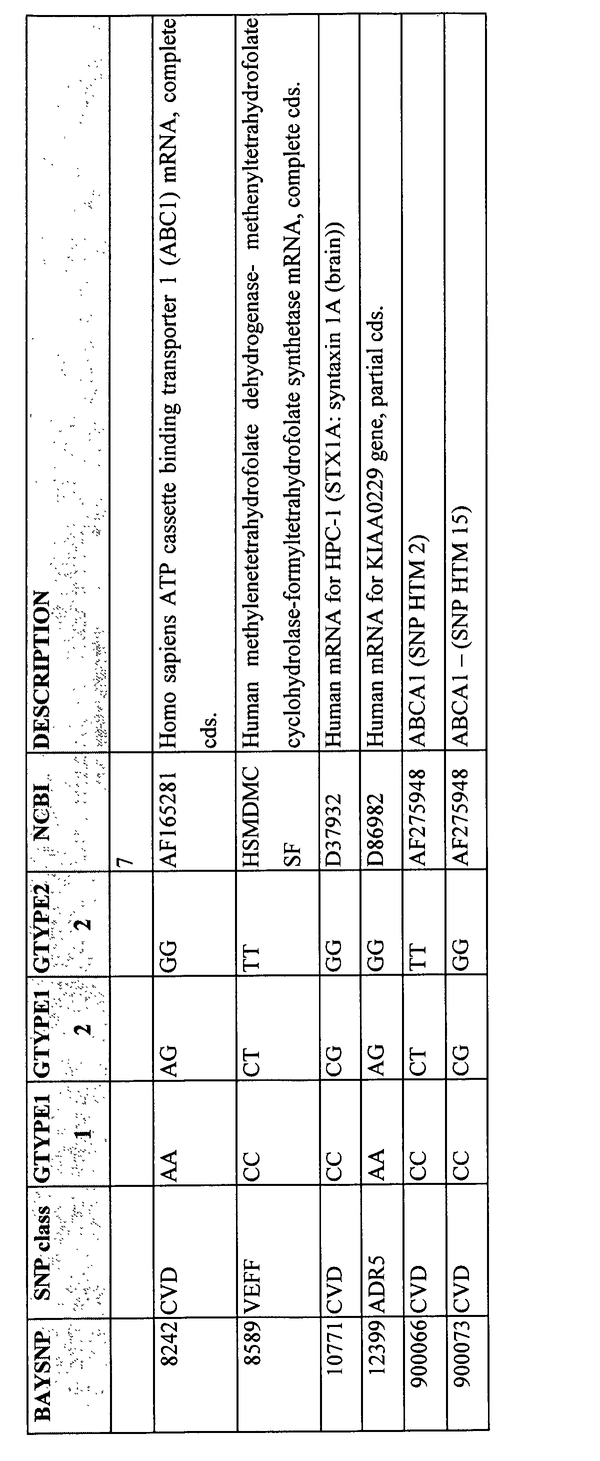

**Table 4**

| **Cohorts** | |
|---|---|
| Given are names (as used in table 5) and formations of the various cohorts that were used for genotyping | |

| **COHORT** | **Definition** |
|---|---|
| HELD_ALL_GOOD/BAD | Healthy elderly individuals of both genders with good or bad serum lipid profiles (as defined in table 1a) |
| HELD_FEM_GOOD/BAD | Healthy elderly individuals (female) with good or bad serum lipid profiles (as defined in table 1a) |
| HELD_MAL_GOOD/BAD | Healthy elderly individuals (male) with good or bad serum lipid profiles (as defined in table 1a) |
| CVD_ALL_CASE/CTRL | Individuals with diagnosis of cardiovascular disease and healthy controls (both genders) |
| CVD_FEM_CASE/CTRL | Individuals with diagnosis of cardiovascular disease and healthy controls (female) |
| CVD_MAL_CASE/CTRL | Individuals with diagnosis of cardiovascular disease and healthy controls (male) |
| HELD_FEM_ADRCTRL | Female individuals that tolerate adminstration of cerivastatin without exhibiting signs of ADR (as defined in table 1b) |
| HELD_FEM_ADRCASE | Female individuals that exhibited ADR (as defined in table 1b) upon administration of cerivastatin |
| HELD_MAL_ADRCTRL | Male individuals that tolerate adminstration of cerivastatin without exhibiting signs of ADR (as defined in table 1 b) |
| HELD_MAL_ADRCASE | Male individuals that exhibited ADR (as defined in table 1b) upon administration of cerivastatin |
| HELD_ALL_ADRCTRL | Individuals of both genders that tolerate adminstrati- |
| | on of cerivastatin without exhibiting signs of ADR (as defined in table 1b) |
| HELD_ALL_ADRCASE | Individuals of both genders that exhibited ADR (as defined in table 1b) upon administration of cerivastatin |
| HELD_FEM_LORESP | Female individuals with a minor response to cerivastatin administration (as defined in table 1b) |
| HELD_FEM_HIRESP | Female individuals with a high response to to cerivastatin administration (as defined in table 1b) |
| HELD_FEM_HIHDL/LOHDL | Healthy elderly individuals (female) with high or low serum HDL cholesterol levels (as defined in table 1c) |
| HELD_MAL_HIHDL/LOHDL | Healthy elderly individuals (male) with high or low serum HDL cholesterol levels (as defined in table 1c) |
| HELD_ALL_HIHDL/LOHDL | Healthy elderly individuals of both genders with high or low serum HDL cholesterol levels (as defined in table 1c) |
| HELD_FEM_ADR3CASE | Female individuals that exhibited advanced ADR (as defined in table 1b) upon administration of cerivastatin |
| HELD_MAL_ADR3CASE | Male individuals that exhibited advanced ADR (as defined in table 1b) upon administration of cerivastatin |
| HELD_ALL_ADR3CASE | Individuals of both genders that exhibited advanced ADR (as defined in table 1b) upon administration of cerivastatin |
| HELD_FEM_VLORESP | Female individuals with a very low response to cerivastatin administration (as defined in table 1b) |
| HELD_FEM_VHIRESP | Female individuals with a very high response to cerivastatin administration (as defined in table 1b) |
| HELD_FEM_ADR5CASE | Female individuals that exhibited severe ADR (as defined in table 1b) upon administration of cerivastatin |
| HELD_MAL_ADR5CASE | Male individuals that exhibited severe ADR (as defined in table 1b) upon administration of cerivastatin |
| HELD_ALL_ADR5CASE | Individuals of both genders that exhibited severe ADR (as defined in table 1b) upon administration of cerivastatin |
| HELD_FEM_ULORESP | Female individuals with a ultra low response to cerivastatin administration (as defined in table 1b) |
| HELD_FEM_UHIRESP | Female individuals with a ultra high response to to cerivastatin administration (as defined in table 1b) |

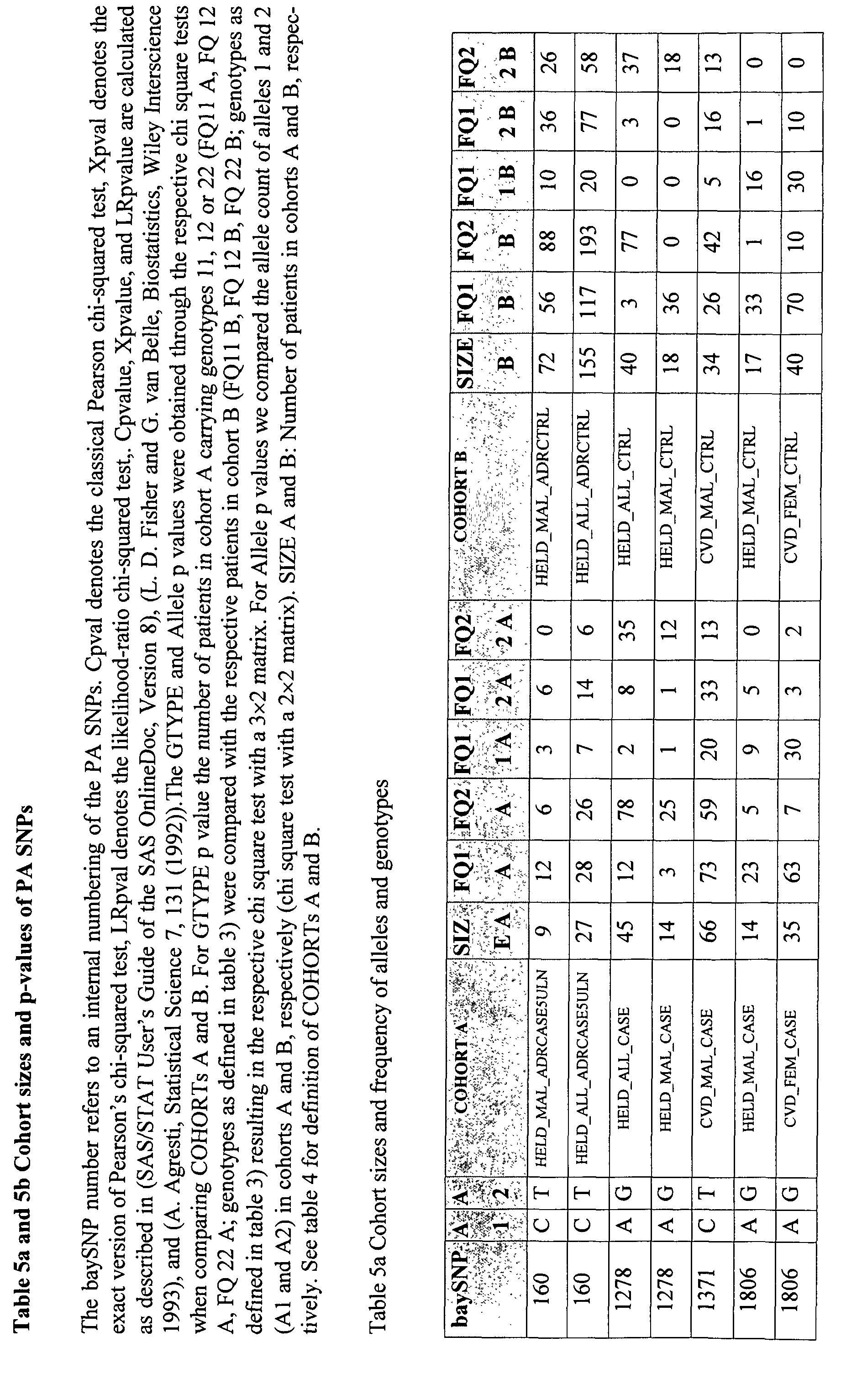

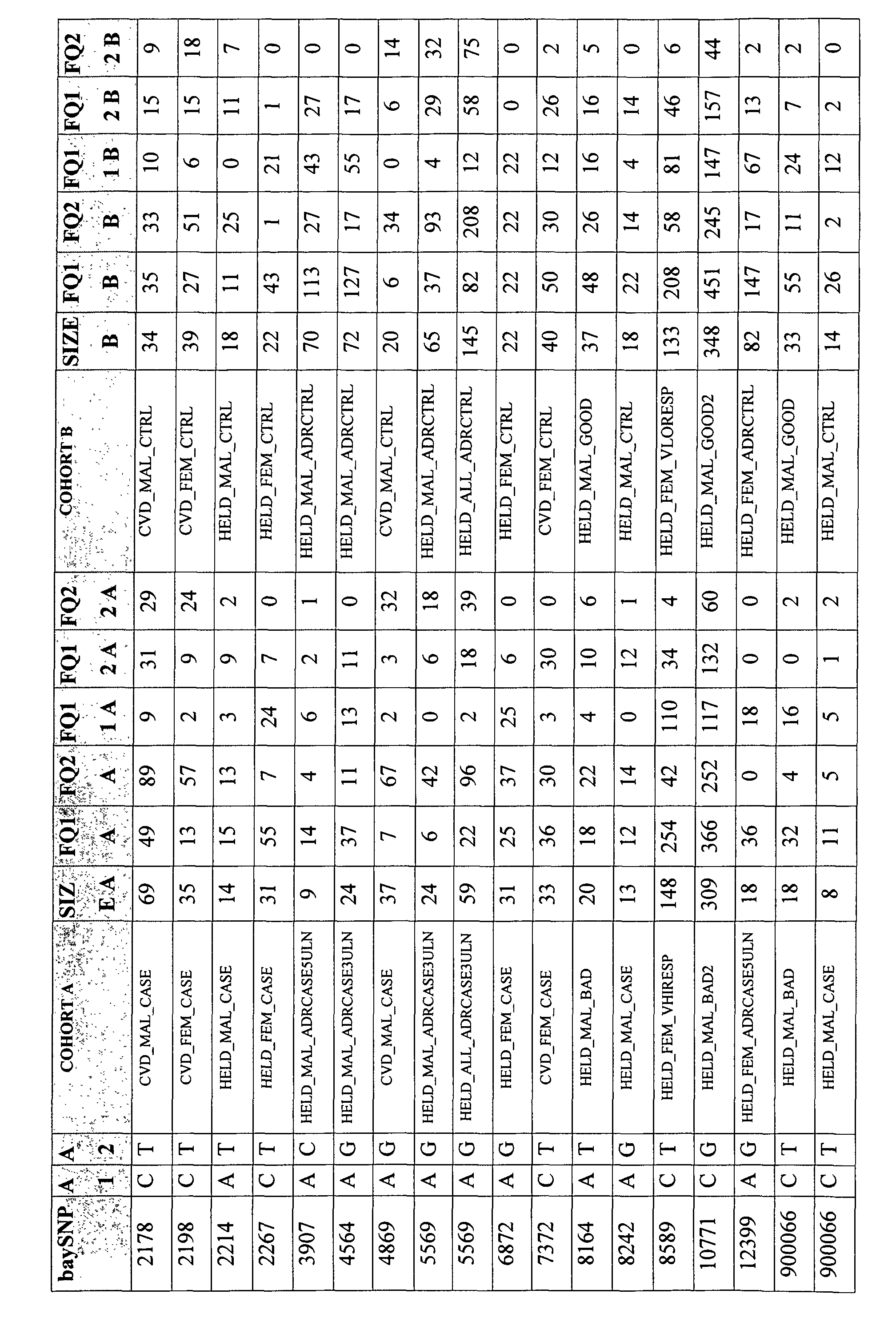

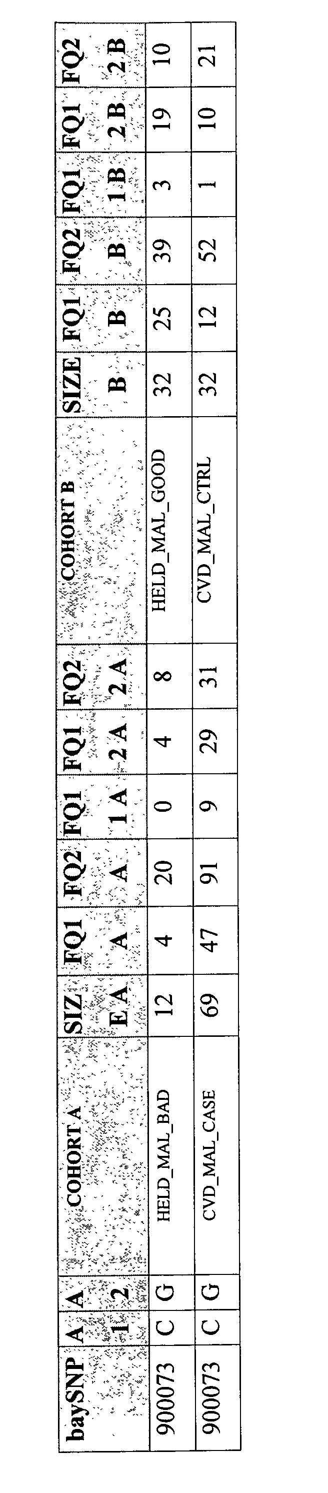

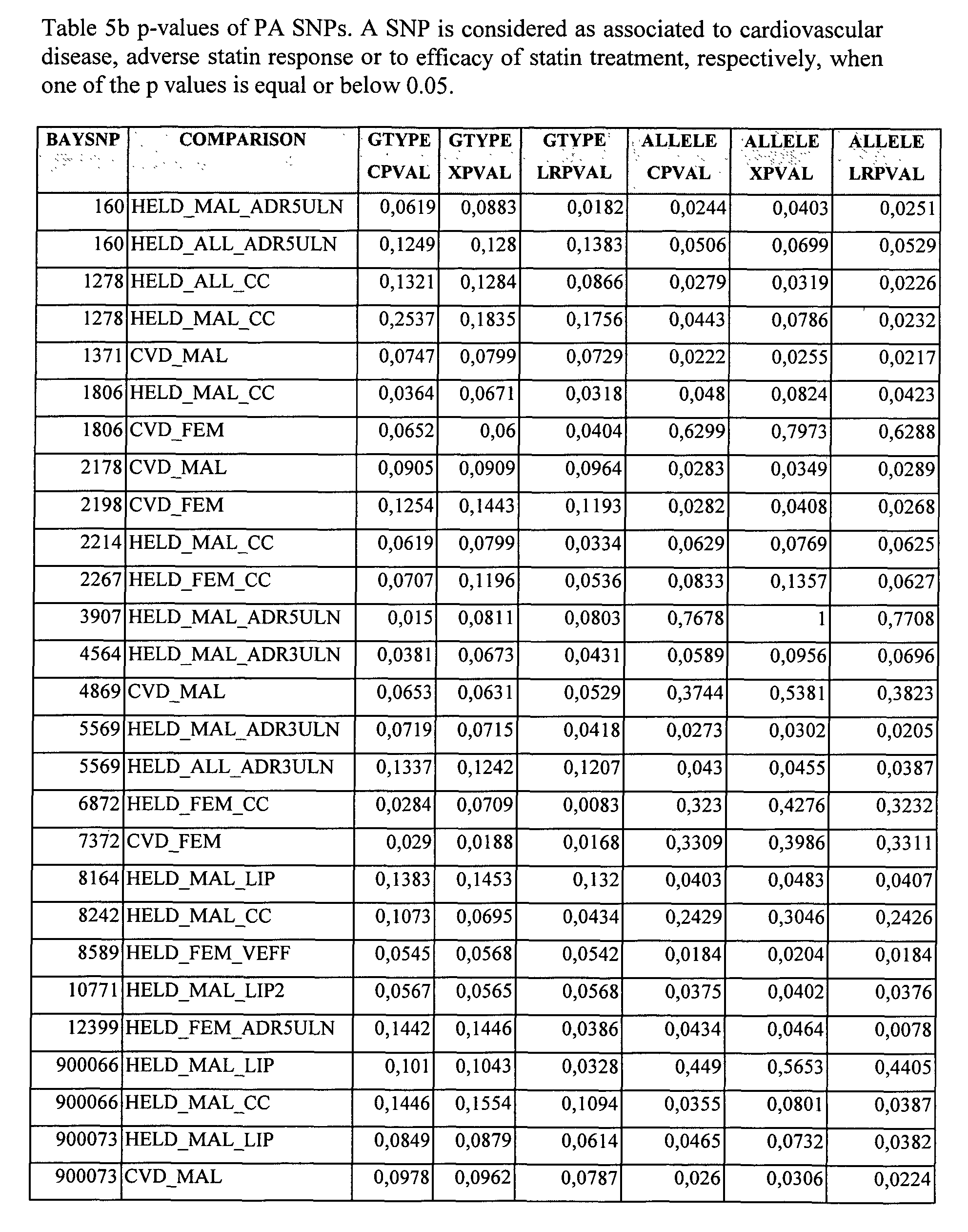

### Table 6a Correlation of genotypes of PA SNPs to relative risk

For diagnostic conclusions to be drawn from genotyping a particular patient we calculated the relative risk RR1, RR2, RR3 for the three possible genotypes of each SNP. Given the genotype frequencies as

| | gtype 1 | gtype2 | gtype3 |
|---|---|---|---|
| case | N11 | N12 | N13 |
| control | N21 | N22 | N23 |

we calculate

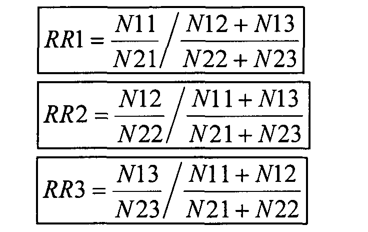

Here, the *case* and *control* populations represent any case-control-group pair, or bad(case)-good(control)-group pair, respectively (due to their increased response to statins, 'high responders' are treated as a case cohort, whereas 'low responders' are treated as the respective control cohort). A value RR1>1, RR2>1, and RR3>1 indicates an increased risk for individuals carrying genotype 1, genotype 2, and genotype 3, respectively. For example, RR1=3 indicates a 3-fold risk of an individual carrying genotype 1 as compared to individuals carrying genotype 2 or 3 (a detailed description of relative risk calculation and statistics can be found in (Biostatistics, L. D. Fisher and G. van Belle, Wiley Interscience 1993)). The baySNP number refers to an internal numbering of the PA SNPs and can be found in the sequence listing. null: not defined.

In cases where a relative risk is not given in the table (three times zero or null) the informative genotype can be drawn from the right part of the table where the frequencies of genotypes are given in the cases and control cohorts. For example BaySNP 12399 gave the following results:

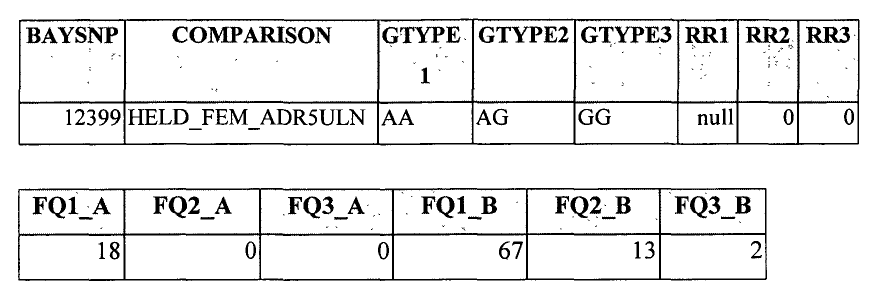

It can be concluded that a AG or GG genotype is only present in the control cohort; these genotypes are somehow protective against ADR. An analogous proceeding can be used to determine protective alleles if no relative risk is given (table 6b).

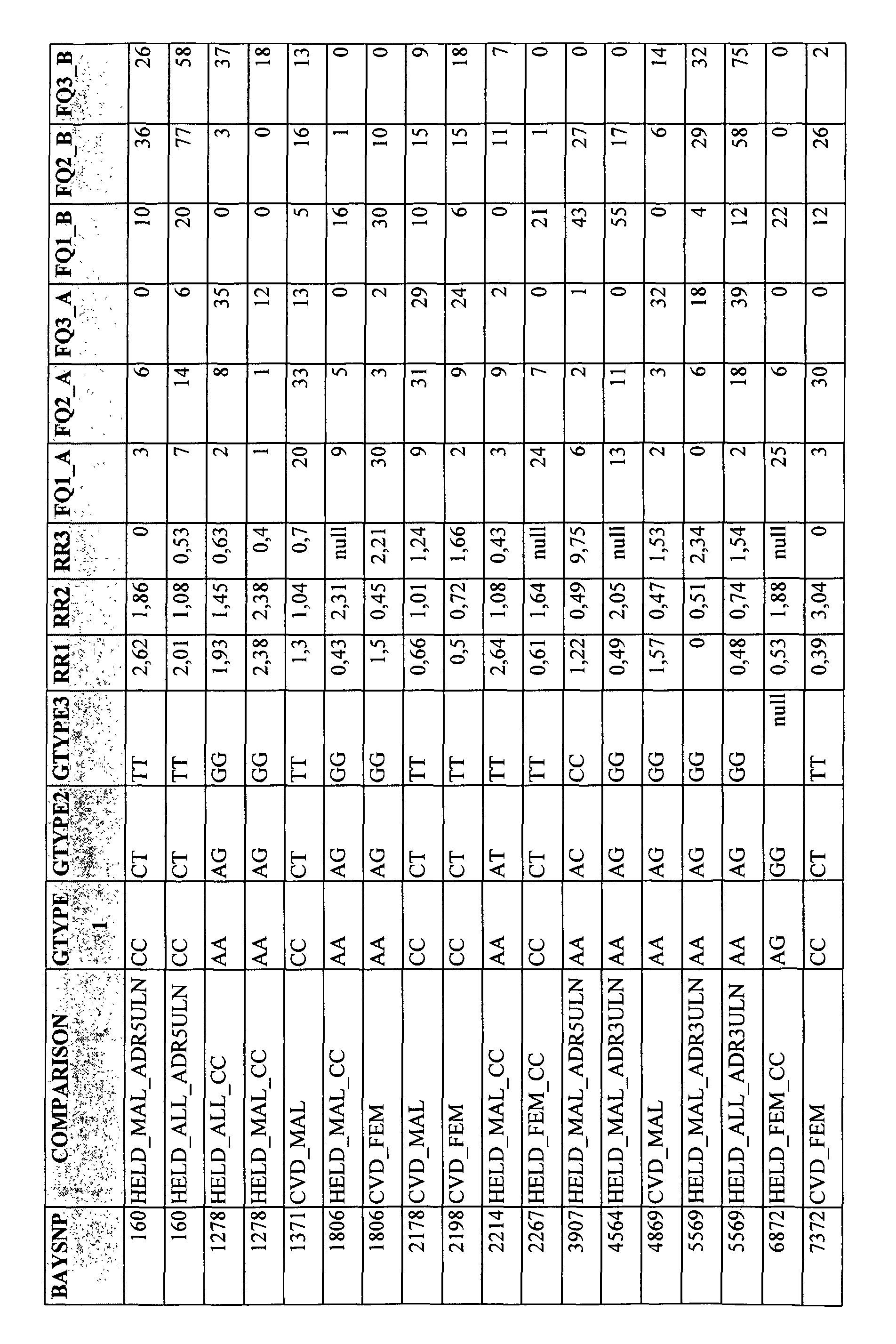

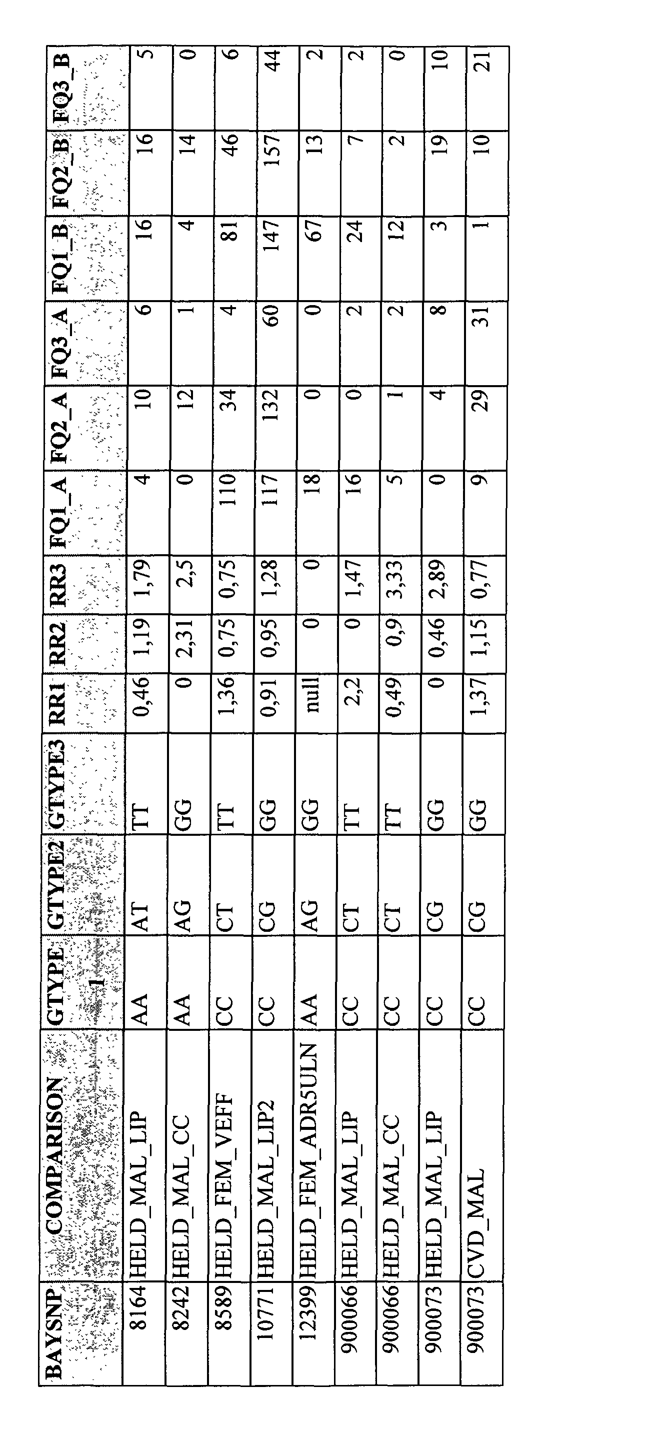

### Table 6b: Correlation of PA SNP alleles to relative risk

For diagnostic conclusions to be drawn from genotyping a particular patient we calculated the relative risks RR1, and RR2 for the two possible alleles of each SNP.
Given the allele frequencies as

| | allele1 | allele2 |
|---|---|---|
| case | N11 | N12 |
| control | N21 | N22 |

we calculate

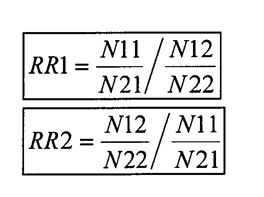

Here, the *case* and *control* populations represent any case-control-group pair, or bad(case)-good(control)-group pair, respectively (due to their increased response to statins, 'high responders' are treated as a case cohort, whereas 'low responders' are treated as the respective control cohort). A value RR1>1, and RR2>1 indicates an increased risk for individuals carrying allele 1, and allele2, respectively. For example, RR1=3 indicates a 3-fold risk of an individual carrying allele 1 as compared to individuals not carrying allele 1 (a detailed description of relative risk calculation and statistics can be found in (Biostatistics, L. D. Fisher and G. van Belle, Wiley Interscience 1993)). The baySNP number refers to an internal numbering of the PA SNPs and can be found in the sequence listing. null: not defined.

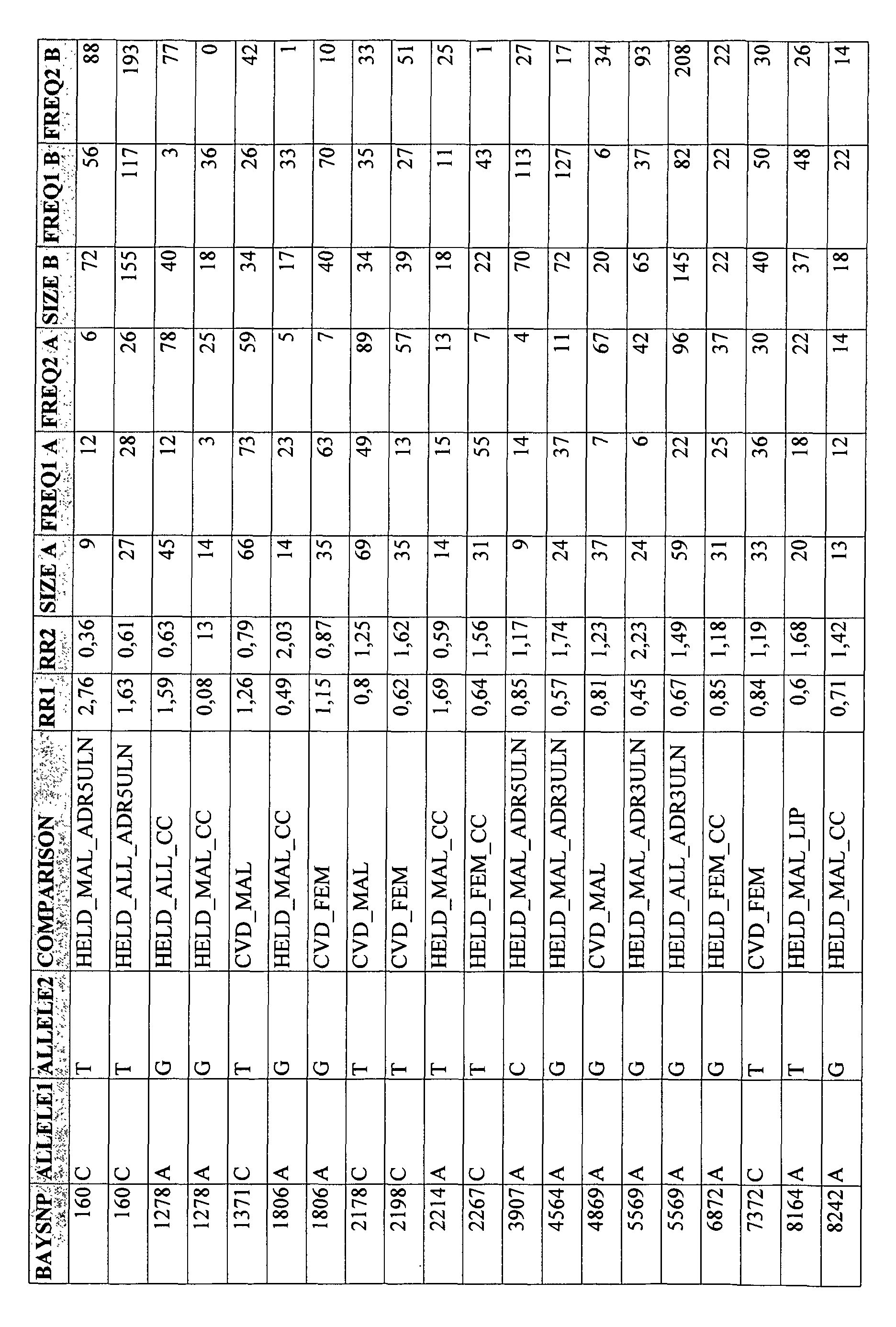

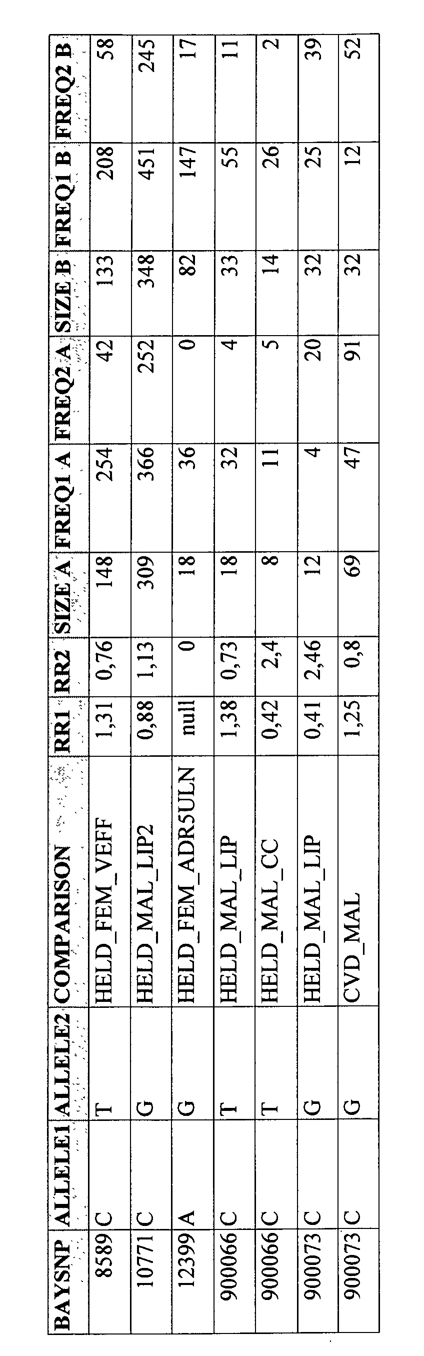

## Claims

1. An isolated polynucleotide encoded by a phenotype associated (PA) gene; the polynucleotide is selected from the group comprising
SEQ ID 1, 2, 3, 4, 5, 6, 7, 8, 9, 10, 11, 12, 13, 14, 15, 16, 17, 18, 19, 20, 21 with allelic variation as indicated in the sequences section contained in a functional surrounding like full length cDNA for PA gene polypeptide and with or without the PA gene promoter sequence.

2. An expression vector containing one or more of the polynucleotides of claim 1.

3. A host cell containing the expression vector of claim 2.

4. A substantially purified PA gene polypeptide encoded by a polynucleotide of claim 1.

5. A method for producing a PA gene polypeptide, wherein the method comprises the following steps:
a) culturing the host cell of claim 3 under conditions suitable for the expression of the PA gene polypeptide; and
b) recovering the PA gene polypeptide from the host cell culture.

6. A method for the detection of a polynucleotide of claim 1 or a PA gene polypeptide of claim 4 comprising the steps of:
contacting a biological sample with a reagent which specifically interacts with the polynucleotide or the PA gene polypeptide.

7. A method of screening for agents which regulate the activity of a PA gene comprising the steps of:#
contacting a test compound with a PA gene polypeptide encoded by any polynucleotide of claim 1; and detecting PA gene activity of the polypeptide, wherein a test compound which increases the PA gene polypeptide activity is identified as a potential therapeutic agent for increasing the activity of the PA gene polypeptide and wherein a test compound which decreases the PA activity of the polypeptide is identified as a potential therapeutic agent for decreasing the activity of the PA gene polypeptide.

8. A reagent that modulates the activity of a PA polypeptide or a polynucleotide wherein said reagent is identified by the method of the claim 7.

9. A pharmaceutical composition, comprising:
the expression vector of claim 2 or the reagent of claim 8 and a pharmaceutically acceptable carrier.

10. Use of the reagent according to claim 8 for the preparation of a medicament.

11. A method for determining whether a human subject has, or is at risk of developing a cardiovascular disease, comprising determining the identity of nucleotide variations as indicated in the sequences section of SEQ ID 1-21 of the PA gene locus of the subject and where the SNP class of the SNP is "CVD" as can be seen from table 3; whereas a "risk" genotype has a risk ratio of greater than 1 as can be seen from table 6.

12. A method for determining a patient's individual response to statin therapy, including drug efficacy and adverse drug reactions, comprising determining the identity of nucleotide variations as indicated in the sequences section of SEQ ID 1-21 of the PA gene locus of the subject and where the SNP class of the SNP is "ADR", "EFF" or both as can be seen from table 3; whereas the probability for such response can be seen from table 6.

13. Use of the method according to claim 12 for the preparation of a medicament tailored to suit a patient's individual response to statin therapy.

14. A kit for assessing cardiovascular status or statin response, said kit comprising
a) sequence determination primers and
b) sequence determination reagents,
wherein said primers are selected from the group comprising primers that hybridize to polymorphic positions in human PA genes according to claim 1; and primers that hybridize immediately adjacent to polymorphic positions in human PA genes according to claim 1.

15. A kit as defined in claims 12 detecting a combination of two or more, up to all, polymorphic sites selected from the groups of sequences as defined in claim 1.

16. A kit for assessing cardiovascular status or statin response, said kit comprising one or more antibodies specific for a polymorphic position defined in claim 1 within the human PA gene polypeptides and combinations of any of the foregoing.
